# EUROPEAN PATENT SPECIFICATION

(11) **EP 3 806 779 B1**
(45) Date of publication and mention of the grant of the patent: **13.12.2023**
(21) Application number: 19819816.0
(22) Date of filing: 17.06.2019
(51) Int. Cl.: A61F 2/24, A61F 2/02, A61B 17/064

(54) **HEART VALVE LEAFLET REPLACEMENT DEVICE AND MULTI-STAGE, MULTI-LUMEN HEART VALVE DELIVERY SYSTEM**
ERSATZVORRICHTUNG FÜR HERZKLAPPENSEGEL UND MEHRSTUFIGES, MEHRLUMIGES HERZKLAPPENFREISETZUNGSSYSTEM
DISPOSITIF DE REMPLACEMENT DE FEUILLET DE VALVULE CARDIAQUE ET SYSTÈME DE POSE DE VALVULE CARDIAQUE À PLUSIEURS ÉTAGES ET À PLUSIEURS LUMIÈRES

(30) Priority: 15.06.2018 US 201862685378 P
(43) Date of publication of application: 21.04.2021
(73) Proprietor: Sutra Medical, Inc., Lake Forest, CA 92630 (US)
(72) Inventor: HAMIDEH, Nur, Marietta, Georgia 30062 (US); PHAM, Thuy, Marietta, Georgia 30062 (US); MARTIN, Caitlin, Marietta, Georgia 30062 (US)
(74) Representative: Avidity IP
(86) International application number: PCT/US2019/037476
(87) International publication number: WO 2019/241777

(56) References cited:
- US-A1- 2009 163 934
- US-A1- 2015 230 924
- US-A1- 2017 258 589
- US-A1- 2017 258 590
- US-A1- 2018 133 007

## Description

This application claims the benefit of U.S. Provisional Application No. 62/685,378, filed June 15, 2018.

### BACKGROUND

The mitral valve (MV) sits between the left atrium (LA) and the left ventricle (LV) of a human heart and normally consists of a mitral annulus (MA), two leaflets, chordae tendineae ("chords"), two papillary muscles, and the left ventricular myocardium. The mitral annulus is subdivided into an anterior portion and a posterior portion. Normally, the anterior mitral leaflet (AML) is connected to the aortic valve via the aortic-mitral curtain, and the posterior mitral leaflet (PML) is hinged on the posterior mitral annulus. The chords originate from either the two major papillary muscles (PPM) or from multiple small muscle bundles attaching to the ventricular wall and connect to the free edge of the mitral leaflets. Chords are composed mainly of collagen bundles, which give the chords high stiffness and maintain minimal extension to prevent the leaflets from billowing into the left atrium during systole. Furthermore, a normal mitral valve consists of right and left trigones, which are two thickened regions that consist of fibrous tissues. The right fibrous trigone is between the aortic ring and the right atrioventricular ring and the left fibrous trigone is between the aortic ring and the left atrioventricular ring.

When the mitral valve is closed, the respective anterior and posterior leaflets are in close contact to form a single zone of apposition. As one skilled in the art can appreciate, normal mitral valve function involves a proper force balance, with each of its components working congruently during a cardiac cycle. Pathological alterations affecting any of the components of the mitral valve, such as chord rupture, annulus dilatation, papillary muscle displacement, leaflet calcification, and myxomatous disease, can lead to altered mitral valve function and cause mitral valve regurgitation (MR).

Mitral regurgitation is dysfunction of the mitral valve that causes an abnormal leakage of blood from the left ventricle back into the left atrium during systole (*i.e.,* the expulsion phase of the heart cycle in which blood moves from the left ventricle into the aorta). While trivial mitral regurgitation can be present in healthy patients, moderate to severe mitral regurgitation is one of the most prevalent forms of heart valve disease. The most common causes of mitral regurgitation include ischemic heart diseases, non-ischemic heart diseases, and valve degeneration. Both ischemic (mainly due to coronary artery diseases) and non-ischemic (idiopathic dilated cardiomyopathy for example) heart diseases can cause functional, or secondary, mitral regurgitation through various mechanisms, including impaired left ventricle wall motion, left ventricle dilatation, and papillary muscle displacement and dysfunction. In functional mitral regurgitation (FMR), the mitral valve apparatus remains normal. Incomplete coaptation of the leaflets is due to enlargement of the mitral annulus secondary to left ventricle dilation and possibly left atrium enlargement. In addition, patients with functional mitral regurgitation can exhibit papillary muscle displacement due to the left ventricle enlargement, which results in excessive tethering of the leaflets. In contrast, degenerative (or organic) mitral regurgitation (DMR) is caused by structural abnormalities of the mitral leaflets and/or the subvalvular apparatus, which can include stretching or rupture of tendinous chords.

The current treatments for mitral valve diseases include surgical repair and replacement of the mitral valve. Mitral valve repair, benefiting from improved understanding of mitral valve mechanics and function, may be now preferred to complete mitral valve replacement. However, the complex physiology and three-dimensional anatomy of the mitral valve and its surrounding structures present substantial challenges when performing these repair procedures.

United States Patent Application publication number US 2017/258589 A1 (Dura Biotech) discloses a heart valve leaflet replacement system and method for same. US 2009/163934 A1 discloses a detachment mechanism for implantable fixation devices. US 2018/133007 A1 (Evalve, Inc.) discloses a cardiac implant delivery system. US 2015/230924 A1 (Valtech Cardio Ltd.) discloses a tissue anchor for annuloplasty device. US 2017/258590 A1 (Middle Peak Medical, Inc.) discloses a device, system, and method for transcatheter treatment of valvular regurgitation.

### SUMMARY

Described herein is a heart valve leaflet replacement system that consists of a heart valve leaflet replacement device (e.g., a prosthetic mitral valve replacement device) and a multi-stage, multi-lumen (MSML) heart valve delivery and implantation system for securing the heart valve leaflet replacement device to one or more of the native valve annuli. The following methods do not comprise port of the claimed subject matter. It is contemplated that the method of securing a heart valve replacement device to one of the native valve annuli is configured to prevent dislodgement of the heart valve device from the annulus. In some embodiments, the MSML heart valve delivery and implantation system can be configured to guide and secure the prosthetic mitral valve replacement device to the native mitral annulus. In some embodiments, the MSMI, heart valve delivery and implantation system can be configured to guide and secure the prosthetic tricuspid valve replacement device to the native tricuspid annulus. In some embodiments, the associated methods can be configured to implant the valve leaflet replacement device to prevent further dilation of the native mitral annulus. For clarity, it can be appreciated that this disclosure focuses on the delivery and implantation of valve leaflet replacement devices for the treatment of functional and degenerative mitral regurgitation, however it is contemplated that the valve leaflet replacement device, the MSMI, delivery and implantation system and the associated methods can be used or otherwise configured to be used to treat other valve disease conditions and replace other valves of the human heart, or could be used or otherwise configured to be used in other mammals suffering from valve deficiencies as well.

In one aspect, the valve leaflet replacement system can comprise a heart valve leaflet replacement device that is configurable or otherwise sizable to be crimped down to fit within a valve delivery sheath and to subsequently be selectively expanded to an operative size and position once removed from the valve delivery sheath within the heart. In some embodiments, at least a portion of the heart valve leaflet replacement device can have a stent shape, which can comprise an upper atrial flared portion and a lower vertical ventricular portion. In some embodiments, the atrial flared portion can be configured to couple with a plurality of dual guiding and fixation (DGF) members to guide and fixate the stent on the annulus, which can help prevent paravalvular leakage and dislodgement of the heart valve leaflet replacement device. Further, the lower ventricular portion can displace a diseased native leaflet out of the blood flow tract and house at least one prosthetic leaflet. In some embodiments, the heart valve leaflet replacement device can comprise a lining skirt that can be coupled to at least a portion of the inner and/or outer surfaces of the stent. In some embodiments, at least one prosthetic leaflet can be mounted on the inner lumen of the stent and/or on at least a portion of the outer side of the stent, which can function in place of at least one native leaflet to restore normal valve function, e.g., to prevent mitral regurgitation.

In some embodiments, at least one leaflet of the heart valve leaflet replacement device can have at least one prong-shaped structure which prevents the prosthetic leaflets from billowing into the left atrium and prolapsing. The at least one prong-shaped structure also acts to reduce prosthetic leaflet stress and facilitate the coaptation with at least one of the native mitral valve leaflets, in order to recreate the competent closure anatomy of a native mitral valve with sufficient leaflet coaptation length and height and proper leaflet angles during systole.

In some embodiments, the valve leaflet replacement system can comprise a MSML heart valve delivery and implantation system that can be used to guide, deploy, and fixate a heart valve leaflet replacement device, and a heart valve leaflet replacement system that is configurable or otherwise sizable to be crimped down to fit within the MSMI, delivery system and to subsequently be selectively expanded to an operative size and positioned once removed from the MSML delivery sheath within the heart.

It should be apparent to one of ordinary skill in the art that various other prosthetic valve replacement devices can also be delivered and implanted using the MSMI, delivery methods described in the present disclosure.

In some embodiments, the delivery of the heart valve leaflet replacement device, or a prosthetic valve, can be conducted using several desired delivery access approaches, such as, for example and not meant to be limiting, a minimally invasive surgical, a trans-septal, a trans-atrial, or a trans-apical approach. In some embodiments, the trans-septal approach can comprise creating an opening in the internal jugular or femoral vein for the subsequent minimally invasive delivery of portions of the heart valve leaflet replacement device, or a prosthetic device, through the superior vena cava, which flows into the right atrium of the heart. In this some embodiments, the access path of the trans-septal approach crosses the atrial septum of the heart, and once achieved, the components of the heart valve leaflet replacement device can operatively be positioned in the left atrium, the native mitral valve, and the left ventricle. In some embodiments, it is contemplated that a main docking sheath can be placed therein the access path to allow desired components of the heart valve leaflet replacement system to be operatively positioned in the left atrium without complications.

In some embodiments, one component of the heart valve leaflet replacement device can comprise a plurality of dual-guiding-and-fixation (DGF) members which can be operatively positioned and implanted at desired locations in the native annulus prior to the delivery of the valve stent component. In some embodiments, the DGF members can guide the subsequent precise positioning and fixate the valve stent. In some embodiments, the plurality of DGF members can help prevent leakage of blood between the operatively positioned prosthesis and the native mitral annulus.

In some embodiments, the DGF members can comprise head, body, and tail components. In some embodiments, the DGF head member can be operatively inserted and embedded into the annular tissue. In some embodiments, the DGF head member can be connected to the DGF body member. In some embodiments, the DGF body member can be configured with a DGF locking member to fixate the valve stent to the native mitral annulus. In some embodiments, the DGF body member can be configured with a DGF engager to engage a DGF delivery mechanism for the insertion of the DGF head member into the tissue, and can be configured to engage valve delivery system for the deployment and fixation of the prosthetic valve. In some embodiments, the DGF tail member can be configured as a flexible component. In some embodiments, the tail portion can be used as a means for precisely guiding and securely maneuvering the prosthetic valve to the native mitral valve annulus.

In some embodiments, DGF head member can have a spiral shape that is about 3-8 mm long and 1-4 mm in diameter.

In some embodiments, DGF body member can be configured with a prosthetic valve fixation mechanism consisting of a plurality of DGF locking units. In some embodiments, the DGF locking units can be configured to engage the prosthetic valve to fixate it in the operative position. In an optional alternative example, the DGF body member fixation mechanism can be configured with an additional DGF locking member which can engage the DGF locking unit to fixate the valve stent in the operative position.

In some embodiments, the DGF body member can comprise a plurality of locking units which can be connected in series by means of a flexible component. This flexible component, in some embodiments, can be made of polymeric, metallic, suture materials, biological materials, and the like. In some embodiments, the flexible component can be straight, curved, single, or double or multiple lines. In some embodiments, the distance between each locking unit could be 0.5-1 mm. These locking units can be separated by, for example, tying a plurality of knots on the flexible component in between these locking units. In some embodiments, if the flexible component is made of metal or plastic or the like, small structures, or bumps, can be welded, molded, or adhered to the flexible component. In some embodiments, the proximal end of the flexible component furthest away from the DGF head member can be configured to connect to the DGF tail member.

In some embodiments, the shape of the DGF locking unit can be conical and tapered where the base has a larger diameter. In some embodiments, the locking unit can be hollow. The locking unit can have an overall height of 0.5 to 2 mm, outer diameters of 0.5 - 0.8 mm (at the tip) and 0.6 to 1 mm (at the base), and/or an inner diameter of 0.08 to 0.2 mm. These locking units enable locking of the prosthetic valve to the DGF body members positioned on the muscular annulus in the operative position.

In another some embodiments, the DGF locking member can be configured to have a conical shape. In some embodiments, the conical locking member can have a base and a plurality of 3-6 deflectable teeth. The base of the lock can have a diameter between 1.8 to 2.5 mm and height between 0.15 to 0.3 mm. The plurality of deflectable teeth can rise from the base and curve toward the center. The thickness of each tooth can be between 0.06 to 0.2 mm. The height of the teeth can be between 0.5 to 3 mm. These dimensions can have +/-15% variances.

In some embodiments, the DGF body members can be configured with a through slot to facilitate the attachment of the DGF locking units and/or the DGF tail members.

In some embodiments, the DGF tail members can be a tether that is configured so that one end of the tether is attached to the DGF body member and the other end of the tether can exit the body. Subsequently, the prosthetic mitral valve can be delivered over the DGF tail members such that the atrial flared portion of the stent can be precisely delivered to the DGF body members embedded in the annulus.

In some embodiments, it is contemplated that the DGF tail members can also serve as a mechanism to precisely guide a plurality of additional DGF locking members to the atrial side of the valve stent in the operative position to lock the prosthetic valve at the location of the DGF body members.

Further in some cases a plurality of DGF locking members can be delivered over the DGF tail members and positioned on top of the upper atrial flared portion of the valve stent in the operative position, immediately following the delivery of the prosthetic valve. In some embodiments, the DGF tail members, can pass through the upper atrial flared portion of the prosthetic valve, and enter the locking member, which is configured to selectively engage the locking units on the DGF body members to fixate the prosthetic valve in the operative position. It is contemplated that the portion of the DGF tail member exiting the locking member can be subsequently removed using a conventional suture-like cutting device or detached from the rest of the DGF members by other means.

In some embodiments, it is contemplated that the DGF tail members can consist of sutures or wires that are looped through a through hole on the DGF body members. In some embodiments, the DGF tail member can be subsequently removed from the DGF body member by pulling one of the two free ends of the DGF tail member until it has been entirely removed.

The MSML delivery system for the heart valve leaflet replacement system can comprise a main deflectable docking system that can house and act as a delivery pathway for the valve delivery system, and the DGF delivery mechanism (DDM) for delivery of a plurality of DGF members, the prosthetic valve, and a plurality of additional DGF locking members.

In some embodiments, the docking system can consist of a steerable sheath with an inner diameter of 24Fr and a docking handle.

The docking sheath can be configured with a compliant distal tip which is capable of bending a maximum of about 180 degrees, a stiff proximal portion, and at least one pull-wire traveling along the sheath length. In some embodiments, bending of the docking sheath distal tip can be controlled by tensioning the pull-wire.

In some embodiments, the docking sheath pull-wire can be attached to a docking handle. The docking handle can be configured with a mechanism to tension the pull-wire in order to bend the distal tip of the docking sheath.

The docking sheath can be configured with a coil supporting structure within the wall to prevent kinking under bending

In some embodiments, the DDM can comprise a steerable outer sheath, a torque-driving shaft, a mechanism to engage the DGF body member engager, and a hollow lumen to accommodate the DGF tail member. In some embodiments, the steerable outer sheath can be configured to bend to precisely position the torque-driving shaft for the optimal DGF member implantation site at the mitral annulus. The torque-driving shaft can be configured to transmit torque from the proximal end of the DDM to the distal end, and comprise a mechanism at the distal end to engage a DGF body member engager, such that turning the torque-driving shaft at the proximal end in a first rotative direction can selectively drive the DGF head member into tissue, and turning it in a second rotative direction can remove the DGF head member from the tissue. The DDM can be configured with a hollow channel to accommodate passage of the DGF tail member to the proximal end of the MSML delivery system.

In some embodiments, the DDM torque-driving shaft can be configured to engage a DGF body member by means of a recessed groove and tie. In some embodiments, the distal tip of the DDM torque-driving shaft can be configured with a recessed groove in which the notch or protrusion on the DGF body member can fit into. The torque-driving shaft can have a hollow structure, such that the DGF member tail can pass through it. The DGF member can be selectively held in place on the distal tip of the DDM torque-driving shaft by tying or otherwise fixing the DGF member tail at the proximal end of the DDM with a DGF tail member grabber, and can be released by releasing the DGF tail member grabber.

Following deployment of the DGF member heads to the native annulus, the DGF member tail trailing outside the body, can be passed through the Expandable Compartmentalization (EC) sheath. Having multiple DGF member tails coming out of the docking sheath may lead to the possibility of tail tangling, which could lead to catastrophic event following the valve delivery. The tangling problem of multiple DGF member tails can be solved by using the EC sheath having multiple lumens for passage of the DGF delivery system and to separate and compartmentalize each of the DGF member tails. The EC sheath can be inserted into the docking sheath during DGF member deployments.

The EC sheath is a tubular structure with one or more hollow lumens, depending on the number of DGF members to be implanted, typically a minimum of three lumens. Once a DGF member is implanted along the annulus or sub-annulus, the DGF tail member can exit the body and come out the proximal end of the docking sheath. Each of the DGF tail members can then be identified and separated using the EC sheath to prevent tangling of the DGF tail members.

In some embodiments, the DGF locking member can be delivered and released using a separate DGF locking member sheath within the MSML delivery system.

It is contemplated that the DGF locking member can be configured to pass a tapered DGF body member locking unit in one direction only. The conical shaped locking member can be delivered with a DGF locking member sheath over the DGF tail member, coupled to the DGF locking units. In some embodiments, when the conical shaped locking member is delivered via a DGF locking member sheath, the locking member can pass through the tapered locking units because the deflectable teeth can be opened by the radial contact force generated by the tapered structure of the locking units. The locking member can be pushed past one or more locking units until the prosthetic valve is in the optimal position. Once the locking member has been pushed past a locking unit, it cannot move back over a locking unit, which can effectively lock the prosthetic valve in position at the annulus. In some embodiments, a DGF locking member sheath can engage the locking member during the forward motion of delivery through the catheter and disengage the locking member when the DGF locking member sheath is retracted.

In some embodiments, simultaneous delivery of multiple DGF locking members can be achieved using a DGF lock housing structure (LHS) within the MSMI, delivery system. In some embodiments, the LHS can be configured with multiple lumens to house multiple locking members and locking member sheaths. In some embodiments, it can be appreciated that the LHS can also be configured to aid in the loading of the prosthetic valve into the MSML delivery system.

Further in some embodiments, one skilled in the art can appreciate that if the spacing between the plurality of implanted DGF head members is larger than the spacing on the DGF tail member receiving holes on the atrial flared portion of the stent, engaging the locking members on the locking units closer to the DGF head members can result in cinching of the posterior mitral annulus.

The MSML delivery system can be configured with a valve delivery system distinct from the DDM. The valve delivery system can be configured to deploy the prosthetic valve and the additional DGF locking members on top of the prosthetic valve.

In some embodiments, the heart valve leaflet replacement device can be delivered to the native mitral annulus from a trans-atrial or trans-septal approach via catheter. The prosthetic valve can be guided to the precise position by a plurality of DGF members configured so that the tail portion passes through the stent and attaches to the head portion of the DGF members previously embedded in the native annulus via a transcatheter approach. Once in the operative position, the prosthetic valve can be locked in place against the annulus at the deployed DGF member bodies by releasing a plurality of locks on top of the atrial flared portion of the stent with the MSMI, delivery system.

In some embodiments, the tail members of the previously implanted DGF members can be configured to pass through specially designed holes on the atrial flared portion of the stent. Alternatively, the DGF tail members can pass through holes in the skirt material of the prosthetic valve.

The valve delivery handle can consist of a sleeve, a valve sheath, a lock housing sheath, a plurality of DGF locking member sheaths, mounting units, and mounting compartments to control the deployment and implantation of the valve stent and DGF locking members.

The valve delivery system can be configured such that the main valve sheath housing the crimped stent goes through the docking sheath. When the prosthetic valve is crimped into a smaller size, it is loaded into the distal end of the valve sheath and is adjacent to the distal end of the LHS system.. A plurality of the DGF locking members and DGF locking member sheaths can be loaded into the distal end of the LHS system, which can allow the DGF locking members to be deployed immediately after the prosthetic valve is released.

The sleeve of the valve delivery system handle consists of the sleeve helix and mounting units. Both the valve sheath and the LHS sheath are permanently fixed to their respective compartments within the sleeve, which controls the movements of the sheaths along the sleeve. During valve releasing, the LHS sheath remains stationary proximal to the crimped stent within the valve sheath. The valve sheath is linked to the sleeve driver which can be moved along the sleeve helix, and releasing the prosthetic valve can be done by rotating the sleeve driver. In some embodiments, the sleeve can be mounted horizontally or at an angle on the platform using the mounting units attached to the distal and proximal ends of the sleeve.

A plurality of DGF locking members are housed within the LHS sheath, which can sit proximal to the crimped stent when loaded in the valve sheath. The position of the DGF locking members within the LHS can be controlled with a plurality of locking member sheaths each connected to a control stud in the DGF locking sheath chamber. In some embodiments, the DGF locking sheath chamber can be configured with a plurality of DGF tail member control studs which can be used to tension the DGF tail members.

In one optional embodiment, the LHS system is coupled to the sleeve to prevent rotation within the handle. Additionally, the valve sheath can be configured such that it may not rotate as it is advanced towards the targeted implant location within the native mitral valve, for example, during delivery. One advantage of this non-rotational feature is that it prevents the DGF tail members from being tangled within the valve delivery system.

Retracting the valve sheath can release the prosthetic valve. In some embodiments, release of the distal end of the stent can be achieved below the annulus. When the distal end of the stent is partially released, the entire valve catheter can be positioned across the valve annulus. The LHS sheaths can be advanced along the DGF member tails immediately following the release of the prosthetic valve to guide the prosthetic valve into position at the deployed DGF members and release the DGF lock members to effectively lock the prosthetic valve in the operative position.

In some embodiments, a valve stabilization mechanism linking the valve stent to the MSML delivery system can be used to keep the valve in position at the annulus following release from the valve sheath such that rapid pacing is not needed during valve release and lock deployment. The valve stabilization mechanism can then be removed after deployment of the locks. One skilled in the art can appreciate that in some embodiments, rapid ventricular pacing may not be necessary, and/or the operator may have more time to deliver the locking members to secure the valve stent in place at the annulus.

It is contemplated that the valve stabilization method can be configured as a looped suture and a tube, where the suture is looped through portions of the valve stent, with the two free ends of the suture exiting through the tube and out of the proximal end of the MSML delivery system. The two ends of suture can be selectively tied to link the prosthetic valve to the MSMI, delivery system, and then the suture can be easily removed from the body by pulling one end from the proximal end of the valve delivery system.

In an optional embodiment, the valve delivery system can also comprise a valve sheath cap which is placed over the distal end of the crimped stent. One skilled in the art can appreciate that it is possible for the DGF tail members tails to get caught on the valve stent during valve release. It is contemplated that the valve sheath cap can be used to cover the ventricular struts on the valve stent to prevent the DGF tail members tails from getting caught under the stent during valve release. Following valve release, the valve sheath cap can be advanced to fully release the valve stent, and then retracted back into the MSMI, delivery system and removed from the body.

In some embodiments, the valve sheath cap can have a cylindrical hollow proximal portion to fit over the ventricular portion of the crimped stent and a rounded dome or conical shaped distal tip. The cylindrical portion can be configured with longitudinal slits to accommodate the DGF member tails, which in operation would trail from the DGF member bodies previously implanted at the native annulus through the valve and lock delivery systems. In some embodiments, the dome or conical shaped tip functions as a nose cone for the valve sheath to aid in navigation of the valve sheath to the native annulus within the body.

In some embodiments, the proximal hollow portion of the valve sheath cap can be configured with a series of collapsible teeth in the shape of a hollow cone pointing proximally which can be opened into a cylindrical shape in order to sheath a portion of the crimped valve stent, and can collapse once the valve stent is released from the valve sheath back to its conical shape. One skilled in the art can appreciate that the conical shape of the valve sheath cap can facilitate retraction of the valve sheath cap back into the MSML delivery system following valve deployment.

In an optional embodiment, the valve stabilization mechanism can comprise a tube to house the suture linking the prosthetic valve to the MSML delivery system which is configured to attach to a valve sheath cap. The valve sheath cap can be configured to cover the lower stent struts on the ventricular portion of the crimped valve stent, such that the DGF member tails do not get caught on the lower stent struts of the valve stent during valve deployment. The valve stabilization mechanism tube can be advanced to push the valve sheath cap towards the ventricle and fully release the valve once the valve sheath has been retracted. Once the locks have been deployed over the valve, the valve sheath cap can be retracted by retracting the valve stabilization mechanism tube back into the docking sheath.

In some embodiments, release of the DGF locking memebrs and DGF locking member sheaths can be achieved by manipulating the two linking structures that are coupled to the lock housing structure and the main delivery system handle. In some embodiments, one linking structure enables simultaneous push out of the locking catheters, and the second linking structure, adjacent to the first linking structure, enables release of the locking devices onto the atrial flared portion of the stent.

In an additional embodiment, release of the DGF locking members can be achieved by manipulating the DGF locking member sheaths from the main delivery system handle only.

It is contemplated that any suture-like cutting device can be used to cut the remaining tails exiting the proximal portion of the locking device. In an alternative embodiment, it is contemplated that the tails can be configured as a loop through the body portion of the DGF members inside the catheter; thus, continuously pulling one end of the tail can decouple the tails from the body portion of the DGF members and remove the whole tail from the patient's body.

In some embodiments, it is contemplated that following deployment of the prosthetic valve and DGF locking members, should there be any paravalvular leak or instability of the valve in the operative position, a plurality of additional DGF members can be deployed on top of the valve with the DDM, such that the DGF head member is driven through the skirt material on the atrial flared portion of the valve stent and embedded into the muscular annulus tissue until the body portion of the DGF body member lies flush against the atrial flared portion of the valve. In some embodiments, no additional fixation mechanism is required on the DGF body member.

It is contemplated that following the implantation of the heart valve leaflet replacement system, the valve delivery system can be removed, and a septal closing device can be inserted through the docking sheath to close up the hole on the atrial septum. Then the entire MSMI, delivery system can be removed from the body.

In some embodiments of aspects provided herein, the body portion of the DGF member is configured to link to the DGF member head and comprises a means for selectively engaging and disengaging a DGF member delivery catheter to embed the DGF head into the annuls tissue, and a fixation mechanism for fixating the prosthetic replacement valve to the implanted DGF members. In some embodiments of aspects provided herein, the body portion of the DGF member can be configured with female screw slots to engage with male screw protrusions on the DGF member delivery catheter; wherein the head of DGF member can be operatively implanted by rotating the DGF member delivery catheter in a first rotative direction. Further, the DGF member can be subsequently separated from the DGF member delivery catheter tip by rotating the DGF member delivery catheter in a second rotative direction that is opposite to the first rotative direction to remove the pins from the slots on the body portion of the DGF member.

In some embodiments of aspects provided herein, the body portion of the DGF member can be configured to define two L-shaped slots that are configured to receive two pins inside the DGF member delivery catheter tip. In some embodiments of aspects provided herein, the fixation mechanism of the DGF member body can be configured as a series of at least one protrusion, or locking unit, designed to pass through a passage hole on the flared annular portion of the stent and allow one-way passage of a locking device. In some embodiments of aspects provided herein, the plurality of locking units is configured as a cone, circular, triangular, or dome shape. In some embodiments of aspects provided herein, the plurality of locking units is configured as a tooth, ridge, or hump shape. In some embodiments of aspects provided herein, the fixation mechanism of the DGF member body can be configured as a single continuous flexible DGF member comprises a series of locking units designed to pass through a passage hole on the flared annular portion of the prosthetic heart valve and allow one-way passage of a locking device.

In some embodiments of aspects provided herein, the body portion of each DGF member further comprises a means for selectively attaching and detaching the DGF member tail. In some embodiments of aspects provided herein, the means for selectively attaching and detaching the DGF member tail from the DGF member body comprises a loop, hook, or ring shape on the DGF member body such that the tail portion of the DGF member can be attached to the body portion by looping the tail through the loop, hook, or ring on the body portion, and then can be selectively removed from the body portion by pulling one end of the tail portion. In some embodiments of aspects provided herein, the tail portion of the DGF member comprises an elongated tether that is configured to guide the prosthetic heart valve to the body of the DGF members in position, and to guide a plurality of locking devices to fixate the prosthetic heart valve in the operative position at the operatively positioned plurality of DGF member bodies.

In some embodiments of aspects provided herein, the tail portion of the DGF member extends from the body portion of the DGF member to the proximal end of the delivery system in operation. In some embodiments of aspects provided herein, the tail portion of the DGF member is configured to be selectively coupled and decoupled from the body portion of the DGF member. In some embodiments of aspects provided herein, the tail portion of the DGF member can be made of, but not limited to, flexible metallic, Nitinol, polymeric, synthetic, biologically derived materials and the like.

In some embodiments of aspects provided herein, the plurality of locking devices is configured to accept passage of the tail portion of the DGF member and pass through the locking units on the body portion of the DGF member in one direction only, and is further configured such that it cannot pass through the prosthetic valve. In some embodiments of aspects provided herein, the plurality of locking devices is configured to be one piece with or attach directly to the flared annular portion of the stent. In some embodiments of aspects provided herein, the plurality of locking devices is configured as a distinct structure from the flared annular portion of the stent. In some embodiments of aspects provided herein, the plurality of locking devices is configured as a hollow conical, cylindrical, or spherical structure with a plurality of teeth rising from a circular base that can open up to allow one-way passage of a specially designed locking unit on the body portion of the DGF member. In some embodiments of aspects provided herein, the base of the locking devices is configured to selectively engage and disengage the lock delivery catheter.

In some embodiments of aspects provided herein, the base of the locking devices is configured with a female slot such that a lock delivery catheter with a corresponding male protrusion can selectively engage and disengage the locking device. In some embodiments of aspects provided herein, the plurality of locking devices is configured as hollow conical, cylindrical, or spherical structure with a plurality of teeth that can selectively be snapped shut to engage with the tail portion of the DGF member or the specially designed locking units on the body portion of the DGF member.

In another aspect, the present disclosure provides a multi-stage and multi-lumen (MSML) delivery system for implanting the prosthetic heart valve for treatment of a diseased heart valve that moves between an open configuration and a closed position to regulate blood flow through the heart valve during a cardiac cycle of a heart; the prosthetic heart valve delivery and implantation system comprising: a means for implanting a plurality of DGF members in the valve annulus; a means for guiding and delivering a prosthetic valve to the operative position at a plurality of implanted DGF members; and a means for fixating and locking the prosthetic valve in the operative position by a plurality of implanted DGF members.

In some embodiments of aspects provided herein, the MSMI, delivery system is configured to first implant a plurality of DGF members, and then the prosthetic valve, followed by the locking devices using the tail portions of the DGF members as a guide. In some embodiments of aspects provided herein, the means for implanting a plurality of DGF members to the valve annulus includes a DGF member delivery catheter configured to implant the head portion of the DGF member in annular tissue. In some embodiments of aspects provided herein, the DGF member delivery catheter comprises: a means for selectively coupling and decoupling to a plurality of DGF members; and a means for housing a plurality of elongated DGF member tail portions. In some embodiments of aspects provided herein, the DGF member delivery catheter is configured to be steerable in multiple directions.

In some embodiments of aspects provided herein, the distal tip of the DGF member delivery catheter is configured with pins designed to selectively engage and disengage an inset on the body portion of the DGF member. In some embodiments of aspects provided herein, the distal tip of the DGF member delivery catheter is configured with screw threads that are designed to selectively engage and disengage complementary grooves on the body portion of the DGF member. In some embodiments of aspects provided herein, the proximal portion of the DGF member delivery catheter is configured to house the tail portion of the DGF member.

In some embodiments of aspects provided herein, the DGF member delivery catheter consists of a plurality of catheters; wherein each is configured to implant a single DGF member. In some embodiments of aspects provided herein, the DGF member delivery catheter is configured to house and implant a plurality of DGF members simultaneously. In some embodiments of aspects provided herein, the means for guiding and delivering the flared annular portion of the stent to the operative position at a plurality of implanted DGF members comprises a DGF member tail configured as an elongated tether or rail that links the prosthetic valve to a plurality of DGF member bodies. In some embodiments of aspects provided herein, the tail portion of the DGF members is configured to attach to the body portion of the DGF members and pass through a designated portion (i.e., passage holes) of the flared annular portion of the stent, such that the plurality of tail portions can guide the flared annular portion of the stent to the plurality of implanted DGF member bodies as the crimped stent and prosthetic leaflets are released from the valve delivery catheter.

In some embodiments of aspects provided herein, there is a lock delivery system within the MSML delivery system that is configured to lock the prosthetic valve in the operative position at a plurality of implanted DGF components, comprising: a plurality of lock delivery catheters; a lock housing structure; and a plurality of lock delivery catheter handles. In some embodiments of aspects provided herein, the lock delivery system is configured such that a plurality of locking devices are deployed simultaneously and immediately after the release of the stent and prosthetic leaflets, and is further configured to be manipulated with the plurality of lock delivery catheter handles.

In some embodiments of aspects provided herein, the lock delivery system is configured such that the plurality of locking devices are deployed individually, and is further configured to be manipulated with the plurality of lock delivery catheter handles. In some embodiments of aspects provided herein, the lock delivery catheter can be selectively coupled to a locking device such that the position of the locking device can be controlled by manipulating the lock delivery catheter, and then can be selectively decoupled from the locking device such that the locking device is left in the body and the lock delivery catheter can be removed from the body at the completion of the implantation procedure. In some embodiments of aspects provided herein, the tip of the lock delivery catheter is configured with an extrusion such that it can engage and keep the locking device in a position, and is configured to disengage the locking device to release the locking device at its final location.

In some embodiments of aspects provided herein, the lock housing structure comprises a plurality of lumens or compartments to hold and organize a plurality of lock devices and DGF member tails to prevent deployment issues. In some embodiments of aspects provided herein, the lock housing structure comprises a linking mechanism to engage a selected portion of the stent; wherein the linking mechanism can aid in the loading and controlled release of the stent and prosthetic leaflets from the valve delivery catheter. In some embodiments of aspects provided herein, the locking device further comprises a delivery system that comprises a DGF member tail control system configured to guide the placement of the flared annular portion of the stent and plurality of locking devices. In some embodiments of aspects provided herein, the DGF member tail control system comprises a plurality of DGF tail member control handles. In some embodiments of aspects provided herein, the plurality of DGF member tail control handles is configured to bind to the proximal end of the tail and can be manipulated to tension the tail such that the tail can act as a rail to guide the delivery of the stent and plurality of locking devices.

In one aspect provided herein is a multi-stage and multi-lumen (MSML) delivery system for implanting a prosthetic heart valve comprising a valve stent for treatment of a diseased heart valve, the MSMI, delivery system comprising: a docking system comprising a docking sheath and a docking controller; a dual-guiding-and-fixation (DGF) system for implanting a plurality of DGF members, the DGF system comprising a DGF sheath, a torque-driving shaft and a DGF controller; wherein the plurality DGF members are configured to couple to a flared portion of the valve stent, and a valve delivery system for releasing and locking the prosthetic heart valve, the valve delivery system comprising a valve sheath, a lock housing structure sheath and a plurality of DGF locking member sheaths, a valve stabilization mechanism, and a valve delivery controller; wherein the docking system, the DGF system, and the valve delivery system are collectively configured to advance a plurality of DGF head members to an operative position, to deliver and implant the plurality of DGF head members to the operative position, and to guide, deliver and fixate the prosthetic heart valve to the operative position.

In some embodiments of aspects provided herein, the DGF sheath comprises a distal portion and a proximal portion, wherein a steerable section is at the distal portion and is configured to have a greater flexibility than the proximal portion; and wherein the DGF sheath further comprises a controller configured to operatively bend the steerable section of the DGF sheath up to 180 degrees. In some embodiments of aspects provided herein, each of the plurality of DGF members comprises a DGF body member and a DGF head member, wherein the torque-driving shaft is configured to fit inside a lumen of the DGF sheath, engage the DGF body member and drive the DGF head member into a targeted tissue; and wherein the torque-driving shaft is configure to bend together with the DGF sheath at the distal end during a DGF member delivery. In some embodiments of aspects provided herein, the torque-driving shaft comprises a distal tip that is configured to engage the engager portion of the DGF body member; wherein each of the plurality of DGF members further comprises a DGF tail member extended from the DGF body member, wherein an inner lumen of the DGF sheath is configured to house the DGF tail member, and wherein the DGF tail member is configured to couple with a grabber at the proximal end of the DGF system to fixate the DGF tail member during the DGF member delivery. In some embodiments of aspects provided herein, the torque-driving shaft is configured to work with a hollow coil, a plurality of twisted wires, or a plurality of cords, wherein each of the hollow coil, the plurality of twisted wires, and the plurality of cords is configured to transmit torque along its length in bent configurations.

In some embodiments of aspects provided herein, each of the plurality of DGF members further comprises a DGF tail member extended from the DGF body member, wherein the DGF system further comprises an expandable compartmentalization sheath with a plurality of lumens to organize the DGF tail member during a DGF member delivery; wherein the inner walls separating the plurality of lumens are flexible, bendable, and collapsible, and are configured to be pushed to either side to allow a passage of other members of the DGF system during the DGF member delivery.

In some embodiments of aspects provided herein, each of the plurality of DGF members comprises a DGF head member, a DGF body member, a DGF tail member, a DGF locking unit, and a DGF locking member. In some embodiments of aspects provided herein, the DGF head member is configured to have a spiral, coil, barb, or hook shape, or other shapes that are configured to engage heart tissues. In some embodiments of aspects provided herein, the DGF body member is configured to link to the DGF head member, wherein the DGF body member comprises (i) an engager for selectively engaging and disengaging a torque-driving shaft configured to embed the DGF head member into an annular tissue, and (ii) a fixation mechanism for locking the prosthetic heart valve to the implanted DGF members. In some embodiments of aspects provided herein, the engager of the DGF body member is comprises a male protrusion, and wherein the male protrusion is configured to engage a recess with a corresponding shape on the distal tip of the torque-driving shaft.

In some embodiments of aspects provided herein, the DGF locking unit is configured to be a hollow, conical shape to allow for the passage of a tether-like material that is used to attach a plurality of locking units. In some embodiments of aspects provided herein, the DGF tail member comprises an elongated tether that is configured to guide the prosthetic heart valve to the DGF body members implanted in position, and wherein the elongated tether extends from the DGF body member to the proximal end of the delivery system. In some embodiments of aspects provided herein, the DGF tail member is configured to attach to or detach from the DGF body member by forming a loop shape at the proximal portion of the DGF body member such that the DGF tail member is looped pass through, and then is selectively removed by pulling one end. In some embodiments of aspects provided herein, the DGF locking member is configured to accept passage of the DGF tail member, and wherein the DGF locking member is configured to pass through the DGF locking units on the DGF body member in one direction only, and the DGF locking member is further configured not to pass through the prosthetic heart valve.

In some embodiments of aspects provided herein, wherein one pair of one locking unit and one locking member are configured to become one locked piece, and the pair of one locking unit and one locking member is configured to attach directly to the flared portion of the valve stent. In some embodiments of aspects provided herein, the DGF locking unit and the DGF locking member are configured as a distinct structure from the flared portion of the valve stent. In some embodiments of aspects provided herein, the DGF locking member is configured to be a hollow conical shape with a plurality of teeth rising from a circular base that is open up to allow one-way passage of a specially designed locking unit on the DGF body member, wherein the plurality of teeth are configured to be snapped shut to engage with the DGF locking units on the DGF body member.

In some embodiments of aspects provided herein, the valve sheath has three sections with different stiffness: a proximal section, a middle section and a distal section; wherein the proximal section is a longer shaft and is stiffer than the middle and distal sections; wherein the middle section is the most flexible among the three sections and is configured to bend up to 180 degrees without kinking; wherein the length of the middle section ranges from 60 to 150 mm; wherein the distal portion is a straight and stiff portion that resists deformation to load a crimped prosthetic heart valve, wherein the length of the distal portion ranges from 15 to 35 mm.

In some embodiments of aspects provided herein, each of the plurality of DGF members comprises a DGF locking member and a DGF tail member, and wherein the lock housing structure sheath comprises a plurality of lumens for separating the DGF locking member and the DGF tail member. In some embodiments of aspects provided herein, the lock housing structure sheath comprises a single lumen, wherein the distal end of the lock housing structure sheath is attached to a lock housing structure (LHS), and wherein the LHS comprises a plurality of lumens for separating the plurality of DGF locking member sheaths.

In some embodiments of aspects provided herein, each of the plurality of DGF members comprises a DGF locking member and a DGF locking unit, wherein the DGF locking member sheath comprises a single lumen for housing the DGF locking member, and wherein the distal end of the DGF locking member sheath is attached to a DGF locking member holder for keeping the DGF locking member in place, and engaged with the DGF locking member sheath until the DGF locking member is deployed over the DGF locking unit.

In some embodiments of aspects provided herein, each of the plurality of DGF members comprises a DGF locking member, and wherein the valve delivery controller comprises a first mechanism (M1) to control the release of the prosthetic heart valve from the valve sheath, a second mechanism (M2) to control the positioning of the lock housing structure sheath, and a third mechanism (M3) to control the DGF locking member sheaths to release the DGF locking member from the valve delivery system and fixate the DGF locking member on the prosthetic heart valve. In some embodiments of aspects provided herein, each of the first mechanism (M1) and the second mechanism (M2) comprises a sleeve of a rigid material comprising a distal section with a helix configuration and a proximal section with a straight cylindrical section; a distal sleeve driver; and three outer compartments linked together, each of the three outer compartments is engaged with the sleeve, wherein the three outer compartments are: a medial hemostasis-tubing housing compartment, a proximal LHS locking compartment, and a valve sheath compartment. In some embodiments of aspects provided herein, the valve sheath compartment comprises a proximal portion and a distal portion, wherein the distal portion is configured to attach the valve sheath, wherein the proximal portion is configured to hold the hemostasis-tubing housing component to prevent leaking, and wherein a flush port is configured proximal to the distal portion and exits the opening on the hemostasis-tubing housing compartment, thereby linked to the hemostasis-tubing housing compartment. In some embodiments of aspects provided herein, the third mechanism (M3) comprises: a lock housing structure sheath compartment comprising a body, a shell, and a cap, wherein a flush port resides within the cap, wherein the shell comprises a knob comprising a horizontal cylindrical body with two ends: a distal end and a proximal end, wherein the distal end comprises a lumen to attach to the lock housing structure sheath, and wherein the proximal end comprises a plurality of lumens to house a plurality of DGF locking sheaths; a DGF locking member sheath chamber comprising a plurality of sliders and a plurality of studs, wherein the plurality of slider is configured for a controlled release of the plurality of DGF locking sheaths, wherein each of the plurality of studs is configured to attach to a member of the plurality of DGF locking sheaths; and a plurality of through lumens for passage of a plurality of DGF tail members; and another through lumen for passage of a valve stabilization mechanism tube.

In some embodiments of aspects provided herein, the valve stabilization mechanism comprises: a valve stabilization mechanism tube running through the length of the valve delivery system, a valve stabilization mechanism tether that is looped through portions of the valve stent and through the valve stabilization mechanism tube to the proximal end of the valve delivery system, and a grabber to selectively fixate the two free ends of the valve stabilization mechanism tether. In some embodiments of aspects provided herein, the valve stabilization mechanism tube comprises a controller configured to adjust the position of the valve stent after the release of the valve stent from the valve sheath. In some embodiments of aspects provided herein, the valve stabilization mechanism further comprises: a valve sheath cap attached to the distal end of the valve stabilization mechanism tube; wherein the valve sheath cap comprises a distal portion and a proximal portion, wherein the proximal portion of the valve sheath cap is configured to fit over the distal portion of the crimped prosthetic heart valve such that the lower ventricular struts of the valve stent are covered during a valve deployment, wherein the distal portion of the valve sheath comprises a hollow passage to pass the valve stabilization mechanism tether; and a rounded or conical distal tip configured to facilitate navigation of the valve sheath. In some embodiments of aspects provided herein, the valve sheath cap further comprises: an expandable and collapsible proximal portion configured to be expanded to fit the crimped valve stent inside, and configured be collapsed following the valve deployment to facilitate the retraction of the valve sheath cap back into the docking sheath for removal.

The invention is a multi-stage and multi-lumen (MSML) delivery system for implanting a prosthetic heart valve, according to the appended claims.

### BRIEF DESCRIPTION OF THE DRAWINGS

A better understanding of the features and advantages of the present subject matter will be obtained by reference to the following detailed description that sets forth illustrative embodiments and the accompanying drawings. The features and components of the following figures are illustrated to emphasize the general principles of the present disclosure. Corresponding features and components throughout the figures can be designated by matching reference characters for the sake of consistency and clarity.
Figure 1 is a perspective view of the MSMI, delivery system pathway from the inferior vena cava and crossing the atrial septum to the native mitral valve.

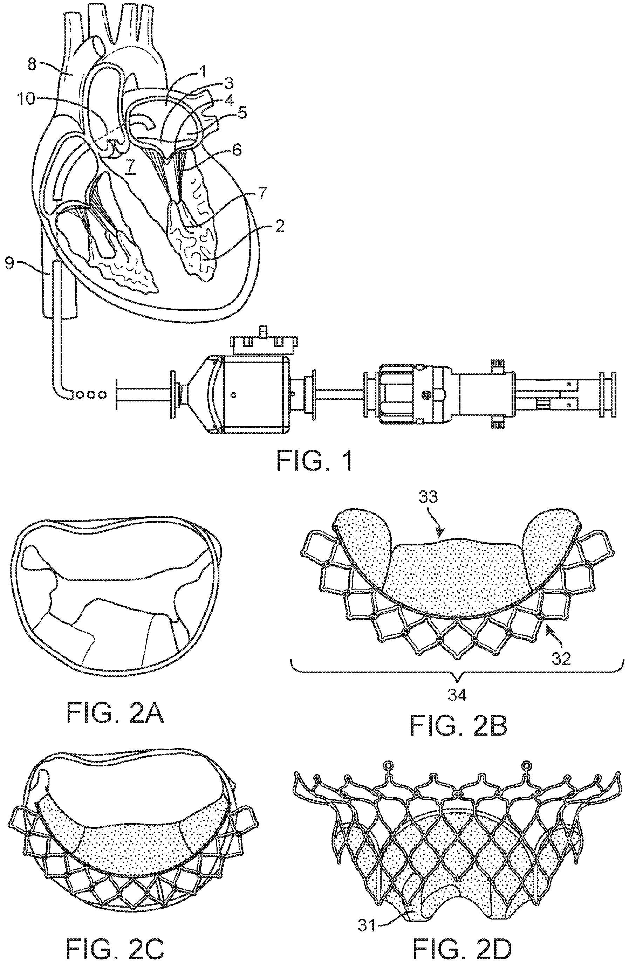

Figures 2A-2C are perspective views of a computer-generated model of an example prosthetic valve leaflet replacement device and stent without a skirt attached for clarity, showing the atrial flared portion and prosthetic leaflets (Fig. 2B) which mimic the native posterior leaflets (Fig. 2A). Figure 2C shows the example prosthetic valve leaflet replacement device in an example operative position in the native mitral annulus (Fig. 2C) with the lateral edges of the stent positioned at the native commissures, such that the prosthetic leaflets can contact the native anterior mitral leaflet under pressurization. Figure 2D shows the example prosthetic leaflet with prong structures.
Figure 3 is a perspective view of a computer-generated model of an example heart valve leaflet replacement system, without a skirt for clarity. The atrial flared portion of the stent is fixated between a plurality of DGF body members sitting below the flared portion of the stent and a plurality of DGF locking units and members sitting on top of the flared portion of the stent.

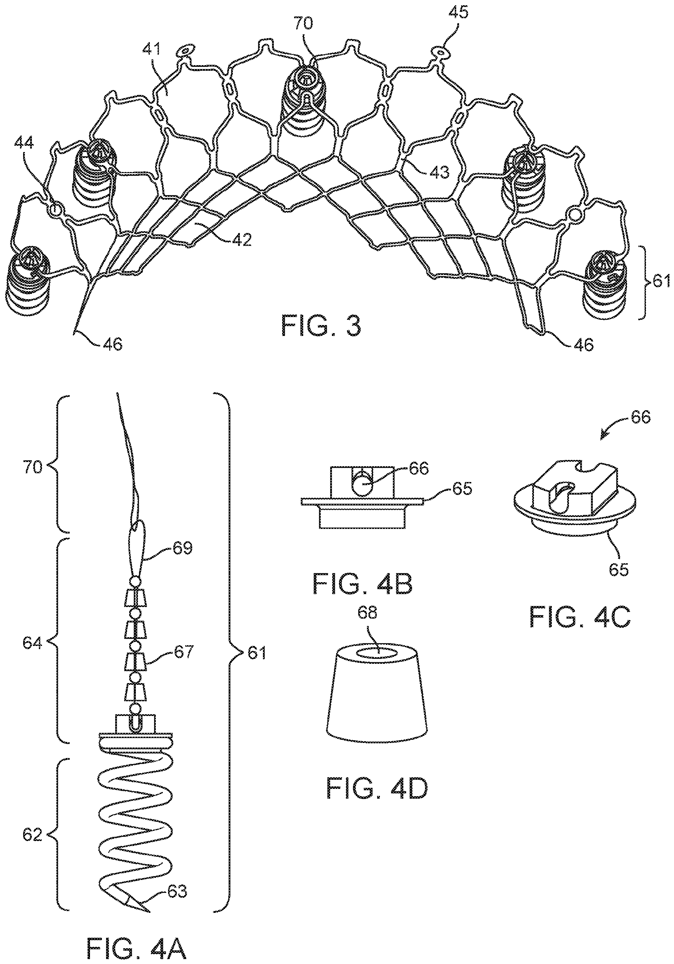

Figures 4A is a perspective view of computer generated models of an example of the DGF member with head, body, and tail portions. The DGF body member, from different views in Figure 4B - 4C, is configured with a through slot to facilitate the attachment of a fixation mechanism consisting of a series of locking units on a tether and an engager component configured as a protrusion to engage with the DDM. The DGF locking unit, as shown in Figure 4D, is configured as a hollow conical shape. The DGF tail member is linked to the DGF body member via the loop at the proximal end of the DGF body member.
Figures 5A - 5B are schematic views of an example DGF locking member.
Figure 6 is a perspective view of the docking system consisting of a docking sheath with a compliant distal tip and a stiff proximal portion, and a docking handle.
Figure 7 is a schematic view of the DDM consisting of a steerable outer sheath with a control knob, a torque-driving shaft with a control knob, and a DGF tail member grabber.
Figure 8 is a schematic view of the DDM within the docking system.
Figures 9A - 9C are perspective views of the distal tip of the torque-driving shaft configured to engage the DGF body members. The distal tip is configured with a slot that is specially designed such that the protrusion on the distal portion of the DGF body member can fit within the slot. Figure 9D is a perspective view of the torque-driving shaft engaged with the DGF body member. The DGF tail member is looped through the through hole on the DGF body member and passed through the hollow lumen of the torque-driving shaft.
Each of Figures 10A - 10C is a perspective view of DGF member deployment steps into the tissue.
Figure 11 is a schematic view of the DDM configured to implant a DGF member into the native annulus.
Figure 12 is a schematic cross-sectional view of the Expandable Compartmentalization (EC) sheath which is configured as a tube with a flexible inner lumen which can be collapsed and pushed to either side.
Figure 13 is a schematic view of the valve delivery system consisting of the valve sheath, LSH sheath, and valve delivery handle.
Figure 14 is a schematic view of the valve delivery system inside the docking system configured to implant the prosthetic valve in the native annulus at the location of the previously implanted DGF members. The crimped valve stent is loaded into the distal tip of the valve sheath. The valve sheath cap covers the distal tip of the valve stent in continuity with the valve sheath.
Figure 15A - 15B are two perspective views of the sleeve within the valve delivery system handle.
Figure 16A - 16B are two perspective views of the valve delivery system sleeve driver. The sleeve driver is configured with ergonomic grips on the outside, a helix on the inside which engages with the helix on the sleeve, and an attachment mechanism to engage with the hemostasis-tubing housing compartment.
Figure 17A - 17B are two perspective views of the hemostasis-tubing housing compartment of the valve delivery system.
Figure 18A - 18B are two perspective views of the LHS locking compartment.
Figure 19A - 19C are perspective views of the valve sheath and valve sheath compartment.
Each of Figure 20A - 20D is a perspective view of the LHS compartment.
Figure 21A - 21B are schematic views of the LHS. The LHS has distal and proximal portions. The proximal portion fits inside the lumen of the LHS sheath, the distal portion fits outside the LHS sheath. There are four lumens: three lumens to house DGF locking members and locking sheaths, and a central lumen for the valve stabilization mechanism.

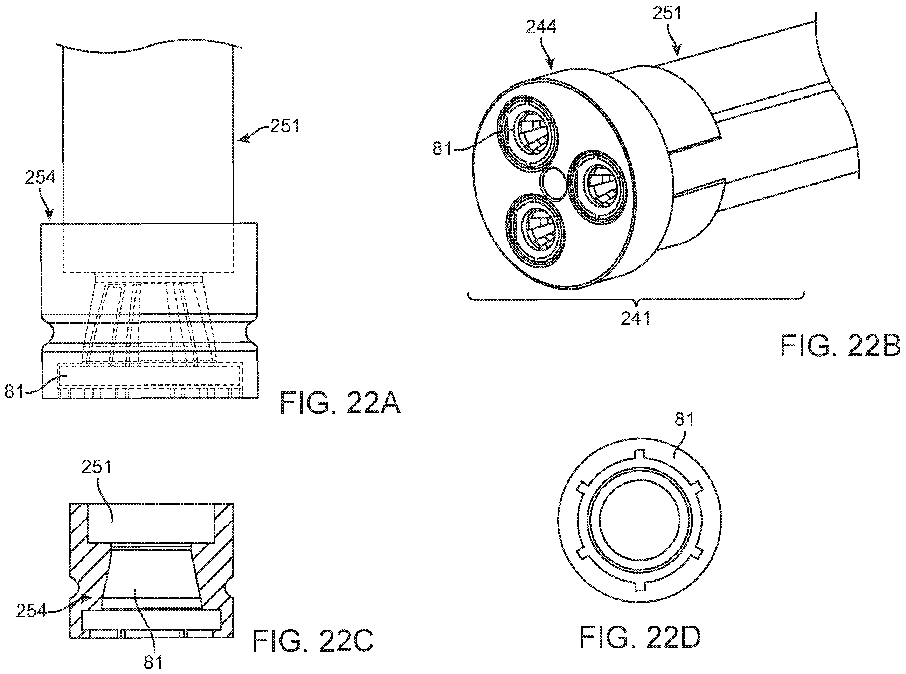

Figures 22A show a schematic view of the DGF locking sheath, the DGF locking member holder and the DGF lock. The distal portion of the locking sheath, as shown in Figure 22C - 22D, is configured with a locking member holder. The locking member holder has a proximal portion, a central portion, and a distal portion. The distal portion, as shown in Figure 22D, has radial protrusions that can keep the DGF locking member inside the locking member holder. Figure 22B shows the LHS configured to house three DGF locking members and locking sheaths simultaneously, the DGF locking member holders can reside inside the lumens of the LHS.
Figure 23A - 23C shows schematic views of an alternative LHS which is configured with five outer lumens to house five DGF locking members and locking sheaths, a central lumen to house a valve stabilization mechanism, guidewire, or other catheter, and includes elements to aid in loading the prosthetic valve into the valve sheath.
Figure 24 is a schematic view showing how the DGF tail members link the DGF head members with the valve delivery system.
Figure 25A is a schematic view of the valve sheath cap. A cross-sectional view of the valve sheath cap, as shown in Figure 25B, illustrates the slots for organizing the DGF tail members. Figure 22C shows another cross-sectional view of the valve sheath cap, showing the lumen to fit the distal end of the valve, a central lumen which provides a means for the valve stabilization mechanism or passage of a guidewire or other catheter, and a distal tip which is rounded or conical in shape to aid in the navigation of the valve sheath within the body.
Figure 26 shows a perspective view of the valve stent with specially configured openings on the lower ventricular portion of the valve stent to accommodate the passage of a valve stabilization mechanism suture.
Figure 27 shows perspective views of the valve sheath cap in usage.
Figure 28A shows a perspective view of the DGF locking sheath chamber, consisting of the sliders and the studs. Figure 28B shows a cross-sectional view of the DGF locking sheath chamber with mounting units on the inlet and outlet, and DGF locking sheath chamber sliders. Figure 28C shows the stud which has an ergonomic shaped head for easy operation.
Figure 29A is a schematic of the first DGF member being deployed at the commissure with the DDM. Figures 29B and 29C show images of an example DGF member being implanted within the posterior mitral annulus in an *ex vivo* pig heart. The left atrium of the heart has been removed for clarity.
Figure 30A - 30F are images of an example of the heart valve leaflet replacement system implantation process in an *ex vivo* pig heart via a 24Fr MSMI, delivery system.
Figure 31 is a schematic view of an example of the heart valve leaflet replacement system deployment process where the MSMI, delivery system is advanced into the left ventricle, and an image of the corresponding step in an *ex vivo* pig heart.
Figure 32 is a schematic view of an example of the heart valve leaflet replacement system deployment process where the prosthetic valve has been nearly completely released from the MSMI, delivery system, and an image of the corresponding step in an *ex vivo* pig heart
Figure 33 is a schematic view of an example of the heart valve leaflet replacement system deployment process where the valve stent has been completely released from the delivery system but has not yet been locked into position, and an image of the corresponding step in an *ex vivo* pig heart.
Figure 34 is a schematic view of an example of the heart valve leaflet replacement system deployment process where the valve stent is locked into position at the implanted DGF body members.
Figure 35 is a schematic view of an example of the heart valve leaflet replacement system deployment process where the DGF tail members are removed from the body once the entire heart valve leaflet replacement system has been implanted.
Figure 36 is a close-up image of an example of the heart valve leaflet replacement system implanted in an *ex vivo* pig heart.

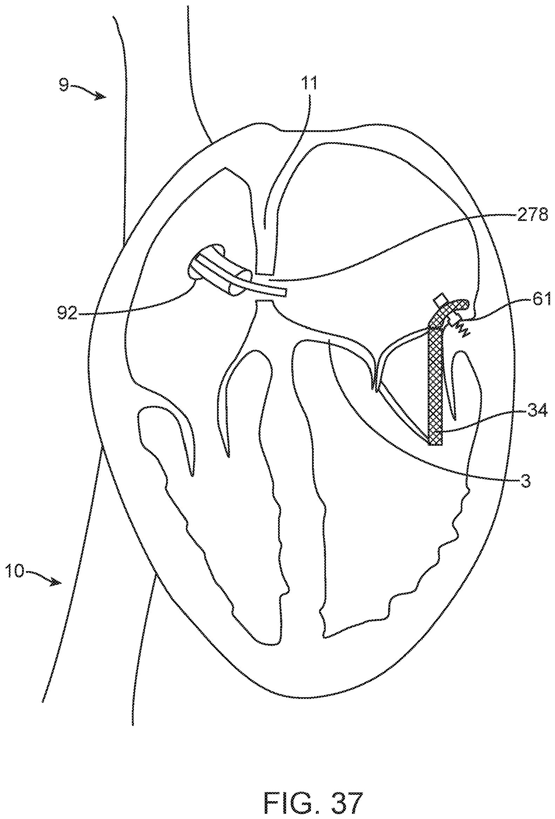

Figure 37 is a schematic view of the MSMI, delivery system being retracted from the body once the heart valve leaflet replacement system has been fixed at operative position at the posterior annulus and the prosthetic leaflets are functioning normally.

### DETAILED DESCRIPTION

The present invention can be understood more readily by reference to the following detailed description, examples, drawings, and claims, and their previous and following description. However, before the present devices, systems, and/or methods are disclosed and described, it is to be understood that this invention is not limited to the specific devices, systems, and/or methods disclosed unless otherwise specified, and, as such, can, of course, vary. It is also to be understood that the terminology used herein is for the purpose of describing some cases only and is not intended to be limiting.

The following description of the invention is provided as an enabling teaching of the invention in its best, currently known embodiment. To this end, those skilled in the relevant art would recognize and appreciate that many changes can be made to the various aspects of the invention described herein, while still obtaining the beneficial results of the present invention. It would also be apparent that some of the desired benefits of the present invention can be obtained by selecting some of the features of the present invention without utilizing other features.

Accordingly, those who work in the art would recognize that many modifications and adaptations to the present invention are possible and can even be desirable in certain circumstances and are a part of the present invention. Thus, the following description is provided as illustrative of the principles of the present invention and not in limitation thereof.

For clarity, it would be appreciated that this disclosure focuses on the treatment of functional mitral regurgitation, however it is contemplated that the heart valve leaflet replacement system and the associated methods can be used or otherwise configured to be used to treat other types of mitral regurgitation or to replace other diseased valves of the human heart, such as tricuspid valve, or could be used or otherwise configured to be used in other mammals suffering from valve deficiencies as well.

As used herein, the singular forms "a," "an" and "the" include plural referents unless the context clearly dictates otherwise. Thus, for example, reference to "a leaflet" can include two or more such leaflets unless the context indicates otherwise.

As used herein, the term "about " refers to an amount that is near the stated amount by about 10%, 5%, or 1%, including increments therein

Ranges can be expressed herein as from "about" one particular value, and/or to "about" another particular value. When such a range is expressed, another aspect includes from the one particular value and/or to the other particular value. Similarly, when values are expressed as approximations, by use of the antecedent "about," it is understood that the particular value forms another aspect. It is further understood that the endpoints of each of the ranges are significant both in relation to the other endpoint, and independently of the other endpoint.

As used herein, the terms "optional" or "optionally" mean that the subsequently described event or circumstance can or cannot occur, and that the description includes instances where said event or circumstance occurs and instances where it does not.

The word "or" as used herein means any one member of a particular list and also includes any combination of members of that list.

The noted challenges to an efficacious mitral valve replacement device generally include operative delivery challenges; positioning and fixation challenges; seal and paravalvular leakage challenges; and hemodynamic function challenges such as left ventricle outflow tract (LVOT) obstruction. With respect to the noted operative delivery challenges, since a conventional mitral prosthesis is larger than a conventional aortic prosthesis, it is more difficult to fold and compress the larger mitral prosthesis into a catheter for deployment as well as retrieval through either conventional trans-apical or trans-femoral delivery techniques.

Turning to the positioning and fixation challenges, instability and migration are the most prominent obstacles given that the mitral valve is subjected to high and repetitive loads in a cardiac cycle, with a high transvalvular pressure gradient that is near zero at diastole and can rise to 120 mmHg or more during systole and higher than 150 mmHg of systolic pressure for patients with aortic stenosis and systemic hypertension. The lack of calcium distribution at the mitral annulus and left ventricular outflow tract (LVOT) obstruction also affect device stability and anchoring. Further, the transcatheter mitral valve replacement can be easily dislodged as the heart moves during each beating cycle.

With respect to sealing and paravalvular leakage, since the mitral valve annulus is large, a good fit between the native annulus and the prosthesis that minimizes paravalvular leak is desirable. Typically, a prosthetic mitral valve may have a large over-hanging atrial portion or flare which can prevent leakage, but, problematically, it also requires a large valve size at the ventricular level so that the prosthesis can be tightly fitted in the native mitral valve. Conventionally, a prosthetic mitral valve is smaller than the diseased native valve and additional material is added around the prosthetic valve to compensate for the large native mitral annulus. Undesirably, adding more material to a prosthetic valve increases the size of the delivery system and may cause valve thrombosis.

Some of the current delivery systems utilize the folding structures of the stent to capture and grab the native leaflets and annulus as a means to anchor the replacement valve. Such methods frequently suffer from the dislodgement of the device due to either insufficient interactive force between the implant and the native tissue, or excess interactive force that leads to native tissue damage (e.g., tearing) and/or remodeling (e.g., elongation, reshaping) which causes the instability and/or migration of the implant.

Finally, with respect to the preservation of hemodynamic function, the operative positioning of a prosthetic mitral valve device, which is conventionally large as described above, should not obstruct the LVOT at the anterior portion of the mitral annulus and should not interfere with the associated structures of a native mitral valve.

Accordingly, it would be beneficial to have a heart valve leaflet replacement device and delivery and implantation system that does not suffer from the shortcomings and deficiencies of current systems. It is desirable to secure the prosthetic mitral valve replacement system to the native mitral annulus. It is also desirable to improve positioning of a mitral prosthesis, avoid LVOT obstruction, and prevent leaking of blood between the mitral prosthesis and the native mitral valve. Similarly, it is desirable to prevent further dilation of the native mitral annulus and allow cinching of the dilated mitral annulus for functional mitral regurgitation patients. Furthermore, other desirable features and characteristics can become apparent from the subsequent detailed description and the appended claims, taken in conjunction with the accompanying drawings and the foregoing technical field and background.

Disclosed are components that can be used to perform the disclosed methods and systems. These and other components are disclosed herein, and it is understood that when combinations, subsets, interactions, groups, etc. of these components are disclosed that while specific reference of each various individual and collective combinations and permutation of these cannot be explicitly disclosed, each is specifically contemplated and described herein, for all methods and systems. This applies to all aspects of this application including, but not limited to, steps in disclosed methods. Thus, if there are a variety of additional steps that can be performed it is understood that each of these additional steps can be performed with any specific embodiment or combination of embodiments of the disclosed methods.

The present methods and systems can be understood more readily by reference to the following detailed description of embodiments and the examples following the description.

Described herein are a heart valve leaflet replacement system 47 and a MSMI, heart valve delivery and implantation system for guiding and securing a heart valve leaflet replacement system 47 to one of the native valve annuli. In some cases, it is contemplated that the heart valve leaflet replacement system 47 and MSML heart valve delivery system can be configured to secure a heart valve leaflet replacement system 47 to the native mitral annulus. In some cases, the heart valve leaflet replacement system 47 and the associated methods can be configured to alleviate mitral regurgitation during a patient's cardiac cycle and/or to help prevent further dilation of the native mitral annulus. It should be noted that it is contemplated that the heart valve leaflet replacement system 47 described herein can be used to replace any diseased valve within the heart. For illustrational purposes, the description in this invention can be focused on the mitral valve and the naming according to the mitral valve geometries. However, the designs described herein can be used in all other heart valves accordingly.

Referring to Figure 1, the mitral valve is located on the left side of the heart, between the left atrium 1 and left ventricle 2 and has anterior 3 and posterior 4 leaflets that are encompassed by the mitral annulus 5. Further, chordae tendineae 6 originate from the respective major papillary muscles 7 of the left ventricular wall and connect to the respective mitral leaflets.

Figure 1 depicts one example of the MSMI, delivery system to deliver a heart valve replacement system 47 to the native mitral annulus. In some cases, the mitral valve can be accessed through a transseptal procedure, where the MSMI, delivery system is inserted in the inferior vena cava 10, enters the right atrium, crosses the atrial septum 11 to the left atrium 1, and then articulates downward to the native mitral annulus 5. It is contemplated that the MSML delivery system could also be navigated to the heart via the superior vena cava 9.

In some cases, a heart valve leaflet replacement system 47 can comprise a replacement prosthetic valve and the means for guiding and fixating the replacement prosthetic valve into the operative position. In some cases, the heart valve leaflet replacement system 47 can be configured to be selectively compressed or otherwise constrained to a compressed position and loaded into the MSMI, delivery system.

Referring to Figures 2A to 2C, the heart valve leaflet replacement system 47 can comprise a crescent shaped stent 32 and at least one mobile prosthetic leaflet 33 which can be configured to replace native leaflet(s) and coapt with the remaining native leaflet(s) in operation. In some cases, the heart valve leaflet replacement system 47 can be configured to replace the native posterior mitral leaflet 4 and coapt with the native anterior mitral leaflet 3.

In some cases, when implanted, it is contemplated that the atrial flared portion 41 of the stent 32 can be configured to be positioned on and/or above the native annulus 5. In some cases, the atrial flared portion 41 of the stent 32 can be configured to facilitate fixation and sealing of the stent, which can assist in preventing paravalvular leakage and dislodgement of the stent post implantation. The mitral valve annulus 5 is asymmetrical, which is illustrated in Figure 1. The atrial flared portion 41 of the heart valve leaflet replacement system 47 can be configured to cover or overlay the posterior portion of the mitral annulus 5, which is divided into three scallops: namely P1, P2 and P3. In some cases, the atrial flared portion 41 can span the two commissures, *i.e.,* the AC-anterior commissure and the PC-posterior commissure. In some cases, the atrial flared portion 41 can comprise an anterior atrial flared portion as well as a posterior atrial flared portion, such that it covers the entire circumference of the mitral valve when operatively positioned.

In some cases, referring to Figure 2, in operative position, at least one prosthetic leaflet 33 is mounted on an inner surface of the ventricular portion 42 of the stent 32. In some cases, at least one prosthetic leaflet 32 of the plurality of prosthetic leaflets has a different shape. In some cases, at least one prong structure 31 comprises a plurality of prong structures. It can be contemplated that the at least one prong structure 31 is coupled to the leaflet free edge.

In some cases of the heart valve leaflet replacement system 47, the prosthetic leaflets 33 can be configured similarly in shape to the native posterior mitral leaflets 4 as depicted in Figure 2.

In some cases, the crescent shaped stent 32 of the heart valve leaflet replacement system 47, as referring to Figure 3, can comprise an atrial flared portion 41, a ventricular portion 42, and neck portion 43 in between. At least a portion of the atrial flared portion 41 and/or a portion of the ventricular portion 42 can be formed to be self-expandable or balloon expandable to the desired operative position. In some cases, it is contemplated that the stent 32 can be conventionally laser cut or woven into a desired stent design that can be radially collapsible and expandable. Thus, it is further contemplated that the stent 32 can comprise a plurality of operatively linked components to form an expandable meshed or non-meshed body that can be made of a metal, or polymeric material, or biologically-made material, including but not limited to, cobalt chromium, stainless steel; or a metal having inherent shape memory properties, including but not limited to, Nitinol. Optionally, it is contemplated that the stent 32 can comprise a plurality of vertical stiff structures that are connected by soft materials such as biological tissue, synthetic materials such as polymers and the like. The stent 32 can be configured to permit the natural dynamic motion of any remaining native leaflet(s) to coapt with the prosthetic leaflet(s) 33.

In some cases, as shown in Figure 3, openings 44 can be defined in the atrial flared portion 41 of the stent 32 can have a circular, square, diamond, triangle, or asymmetrical shapes. The area of the openings 44 of the atrial flared portion 41 can have an area range from about 0.2 mm² to 2 mm²

In some cases, at least a portion of the atrial flared portion 41 has an upward curl to prevent the erosion of the stent into the annulus 5 tissue. In some cases, the upward curl portion is oriented between 100 to 120 degrees from the atrial flared portion 41. In some cases, at least a portion of the atrial flared portion 41 is configured to have a plurality of passage holes 44 to selectively engage the DGF members 61. In some cases, at least a portion of the atrial flared portion 41 is configured to have a plurality of tabs 45 to facilitate the loading and crimping the stent 32 into the valve sheath 201. In some cases, at least a portion of the atrial flared portion 41 is configured with a binding mechanism 46 on the lateral edges such that the lateral edges of the partial circumference frame 32 can be adjoined to make a cylinder to facilitate crimping of the prosthetic valve 34.

Referring to Figure 3, in some cases the heart valve leaflet replacement system 47 is configured with a plurality of DGF members 61 to aid in guiding and fixation of the stent 32. In some cases, the atrial flared portion 41 of the heart valve leaflet replacement system 47 can be guided into position at the posterior portion of the mitral annulus 4 by first implanting a series of DGF members 61 in the annulus.

In some cases, as shown in Figures 4A - 4B, the DGF members 61 have a head portion 62 which engages the tissue on or around the annulus 5, a body portion 64 with an engager 65 that engages a DGF delivery mechanism (DDM) catheter and contains a fixation mechanism, and a tail portion 70 that is configured with a linking mechanism with the stent 32, and in operation, guides the atrial flared portion of the stent 41 to the implanted DGF body member body locations, and a fixation mechanism 85 to lock the implanted valve stent in the operative position.

In some cases, as shown in Figures 4A - 4C, each DGF member 61 can comprise a DGF head member 62, a DGF body member 64, and a DGF tail member 70. It is further contemplated that the DGF head member 61 can be configured to implant into the native annulus tissue and to resist separation after implantation. In some cases, the DGF head member 62 can have, but is not limited to, a spiral shape, a coil shape, a pronged shape, a screw shape, and a barbed hook shape that engage the annular tissues. The DGF head member 62 can be formed of, but are not limited to, Nitinol, stainless steel, cobalt chromium, polymer, and the like. A plurality of DGF members 61 can be sequentially or simultaneously delivered and implanted at the desired locations via a DDM 101. In some cases, it is contemplated that a method of implanting DGF members 61 can be implemented prior to the delivery of the prosthetic valve 34. In some cases, a plurality of DGF members 61 can be configured to help prevent leakage of blood between the operatively positioned heart valve leaflet replacement system 47 and the native mitral annulus 5. In some cases, a plurality of DGF members 61 can be configured to facilitate cinching of the diseased annulus 5 in the circumferential direction to reduce the annular dimensions.

It is contemplated that in some cases, the DGF members 61 may comprise a plurality of additional DGF locking members 81. In some cases, the atrial flared portion of the stent 41 can be locked into position at the implanted DGF body members 64 with a plurality of DGF locking members 81. In some cases, the atrial flared portion of the stent 41 can be locked into position at the implanted DGF body members 64 with a plurality of DGF locking members 81. In some cases, the DGF tail member 70 is also configured with a linking mechanism with the DGF locking members 81 which, in operation, guides the locking members 81 towards the stent 32 and DGF body members 64, thereby sandwiching the atrial flared portion 41 of the stent 32 between the DGF head members 62 and locking devices 81 as shown in Figure 3.

In some cases, the plurality of DGF members 61 can be directly attached to the stent 32. In some cases, it is contemplated that the prosthetic valve 34 can be deployed simultaneously with the DGF members 61 or a portion of the DGF members 61 such as the locking devices 81, and the DGF members 61 can be selectively configured to engage the native annulus 5 once the atrial flared portion of the stent 41 is in the operative position to fasten the device to the native annulus 5.

As one skilled in the art can appreciate, the DGF members 61 in this invention are specially configured to safely secure the prosthetic valve 34 on the muscular annular 5 section of the mitral valve. The DGF head member 62 and body member 64, as shown in Figure 4A, are configured to withstand the total force exerted to the prosthetic leaflet 33, without being pulled off the muscular portion of the annulus 5.

It is contemplated that the DGF member 61 configuration in this invention, as shown in Figure 4A, can withstand dislodging forces imposed by hemodynamic loading of the heart. In some cases, one DGF member 61 can be placed at the posterior commissure and the other DGF member can be placed at the anterior commissure. Optionally, additional DGF members 61 may be placed between these two around the posterior annulus. It is contemplated that the height of the DGF body member 64 can be between about 1 and about 10 mm, and the length of the DGF head member 62 can be between about 3 and about 8 mm. In some cases, as shown in Figure 4A, the DGF head member 62 can have a coiled shape with between about 3 to 6 coils. In some cases, it is contemplated that the coiled DGF head member 62 can have a wire diameter of 0.3 to 1 mm, and the formed outer diameter of the coiled head portion 39 can be from about 2 to about 5 mm.

In some cases, the tip 63 of the DGF head member 62, as shown in Figure 4A, can be shaped and configured to facilitate easy penetration into the annular tissue. In some cases, the tip 63 of the DGF head member 62 is curved to the same pitch as the remainder of the DGF head member 62. In another some cases, the tip 63 of the DGF head member 62 can be straight. Optionally, the tip 63 of the DGF head member 62 can have an arc length of about 1 mm to 3 mm.

In some cases, as shown in Figures 4A - 4B, the DGF body member 64 can define a slot 66 that is configured for attachment of a fixation mechanism and/or the DGF tail member 70. It is contemplated that the DGF tail member 70 and/or a fixation mechanism can be looped through the slot 66 on the DGF body member 64. Optionally, the DGF body member 64 can consist an engager 65 configured as a protrusion to engage a DGF delivery mechanism (DDM) 101.

Referring to Figure 4A, it is contemplated that the DGF head member 62 and body members 64 can be formed as a single component or optionally, by joining distinct parts by means of welding, adhesives, or the like that can resist separation during in vivo loading. Further in some cases, the DGF head member 62 and body members 64 are formed with strong and biocompatible materials such that they can be permanently implanted in the human body and resist damage, for example, but not limited to, stainless steel, cobalt chromium, nitinol, non-absorbable polymer, biological material and the like.

As shown in Figures 4A - 4B, it is contemplated that the DGF body member 64 can be configured with a means for locking the stent 32 in the operative position at the implanted DGF members 61. The locking mechanism can comprise at least one DGF locking unit 67, including but not limited to, ridge engaging teeth, barbs, zip ties, pliable barb or key element, a cone shape, a square shape, an arrow shape, a circular shape, a triangular shape, a dome shape, and the like. In some cases, it is contemplated that the DGF locking units 67 can be configured to allow passage of a portion of the atrial flared portion 41 of the stent 32, guided by the DGF tail member 70, and to resist the subsequent movement of the atrial flared portion 41 of the stent 32 in the opposite direction. In some cases, it is contemplated that the DGF locking units 67 can be configured in series along a tether on the DGF body member 64, as shown in Figure 4A, to allow for adjustment of the distance between the atrial flared portion of the stent 41 and the DGF body member 64, and allow the stent 32 to be operatively locked even when there is a misalignment between the tail receiving holes 44 in the atrial flared portion of the stent 41 and DGF members 61. In some cases, it is contemplated that the one or more DGF locking units 67 can be formed of, but are not limited to, polymers, polytetrafluoroethylene (PTFE), metallic materials, or a combination of these materials.

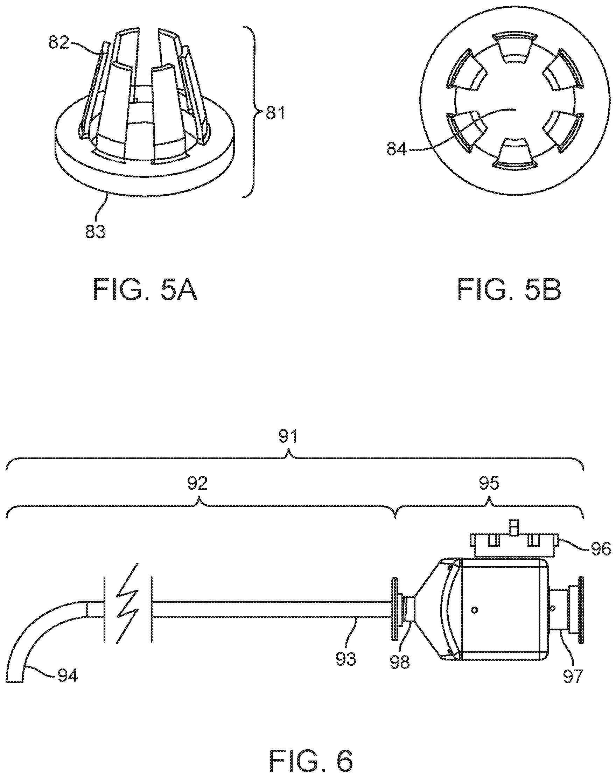

In some cases, a means for locking the atrial portion of the stent 41 to the DGF members 61 is configured to the DGF body member 64 as depicted in Figures 4A - 4B. In some cases, the DGF body member 64 can be configured as two distinct parts: a distal portion linking the DGF body member 64 to the DGF head member 62 and engaging the DDM 101 with an engager 65, and a proximal portion that comprises a fixation mechanism. It is contemplated that the proximal portion of the DGF member body 64 can be configured to link to the distal portion by means of a through hole 66 on the DGF body member 64, and can contain a series of conical shaped DGF locking units 67 specially designed to allow one-way passage of an additional DGF locking member 81, as shown in Figure 5A - 5B. Further in some cases, the proximal portion of the DGF body member 64 can contain a means for linking to the DGF tail member 70. It is contemplated that in some cases, the tether portion of the DGF body member 64 would remain permanently in the patient with the rest of the DGF body member 64 as well as the DGF head member 62, while the DGF tail member 70 can be configured to be removed from the body after the prosthetic valve 34 and plurality of DGF locking members 81 have been implanted. It is contemplated that the proximal portion of the DGF body member 64 can be configured with a loop 69, such that in operation, the DGF tail member 70 can be looped through it, and the two ends of the DGF tail member 70 exit the patient's body. In this some cases, it can be appreciated that the DGF tail member 70 can be easily removed from the patient's body by pulling one of the free ends of the DGF tail member 70 exiting the patient's body, once the implantation procedure is complete.

It is contemplated that the DGF locking units 67 on the DGF body member 64 can be configured to remain in the body. In some cases, the DGF body member can be about 1 to 6 mm in length.

It is contemplated that the DGF tail member 70 can be configured to be long enough to extend from the DGF body member body 64 and through the MSML delivery system. In some cases, the DGF tail member 70 can be about 0.5 to 1.5 m in length.

Referring to Figure 4A, the DGF locking units 67, can be about 1 to 3 mm in length and 1 to 2 mm in diameter. It is further contemplated that the conical locking units 67 can be configured with a through hole 68 such that a tether can pass through, where the tether is configured as a means to attach the conical DGF locking units 67 to the distal portion of the DGF body member 64. In some cases, the DGF locking units 67 can be separated from each other on the tether by knots or other protrusions. It is contemplated that the locking units 67 be formed of a strong, biocompatible material such that they can be permanently implanted in the body and can withstand in vivo loads, for example, but not limited to stainless steel, cobalt chromium, nitinol, non-absorbable polymers, and the like.

In some cases, a portion of the DGF tail member 70 adjacent to the DGF body member 64 can have at least one DGF locking unit 67 that is configured to facilitate locking of the DGF tail member 70 within the DGF locking member 81 and thereby locking the prosthetic valve 34 in the operative position. In some cases, each locking unit 67 can be formed by a knot in the tail material, or optionally by adding additional material to form a male protrusion. The DGF tail member 70 can be formed of, but not limited to, polymers, polytetrafluoroethylene (PTFE), metallic materials, or a combination of these materials.

It is further contemplated that the DGF tail member 70 can be configured to help position and secure the atrial portion of the stent 41 to the implanted DGF body members 64. Optionally, it is contemplated that the DGF tail member 70 can be coupled to the body portion of the DGF body member 64 and can be long enough to extend through the atrial portion of the stent 41 within the MSML delivery system, and outside the patient's body. In some cases, as the crimped stent 32 is released from the MSMI, delivery system, the atrial portion of the stent 41 can be guided to the DGF head members 62 via the DGF tail members 70.

It is contemplated that the atrial portion of the stent 41 can be fixed in the operative position at the location of the implanted DGF head members 62 with a plurality of DGF locking members 81 which are guided into position with the DGF tail members 70. In some cases, the locking member 81 can comprise a structure with a passage 84 that allows at least one DGF tail member 70 to pass through. In some cases, the locking member 81 can be manipulated to be locked within a designated portion of the DGF body member 64.

In some cases, as depicted in Figures 4A - 4B, the DGF member body 40 comprising a plurality of locking units 45 can optionally be configured with a loop at the distal end 87, where the DGF member tail can be looped through and later removed from the patient's body after delivery of the stent 31 and DGF locking devices 70.

In some cases, the DGF locking device 81 can be configured with a conical or dome shape with a plurality of locking teeth 82, specifically designed to pass over a plurality of DGF locking units 67 or male protrusions on the DGF body member 64 in one direction. In some cases, as shown in Figures 5A - 5B, the proximal portion of the DGF locking member 81 can have a flat circular surface 83 that serves as a contact surface for a DGF locking member sheath 251, and six teeth 82 creating a conical shape. Further in some cases, the DGF locking units 67 connected to the DGF body members 64 can have a corresponding conical shape. In operation, when advanced, the DGF locking member sheaths 251 can selectively contact the base of the DGF locking member 83 and push the locking member 81 over one or more locking units 67. In operation, once the locking member 81 is pushed past a locking unit 67, it cannot move back over it in the opposite direction. One can appreciate that once the locking member 81 passes through a locking unit 67, it can prevent motion of the atrial flared portion of the stent 41 hence locking it in the operative position at the implanted DGF head member 62.

In some cases, the DGF locking member 81 can be configured to be selectively locked at any preferred location along either the DGF tail member 70 or the DGF body member 64 to allow for marginal errors in the placement of the DGF members 61 on the annulus 5.

It is contemplated that the heart valve leaflet replacement system 47 can be delivered and implanted in the native mitral annulus through a docking system 91 where the docking system can first be inserted into the body and positioned across the atrial septum 11 to provide access to the left atrium 1. In some cases, the distal tip of the docking sheath 94 can stay in place in the left atrium 1 throughout the entire implantation procedure. The DDM 101 and the valve delivery system 131 are then inserted within the docking system 91 to deliver and implant the DGF members 61, prosthetic valve, and DGF locking members 81.

Referring to Figure 6, in some cases, the docking system 91 consists of a docking sheath 92 with a compliant distal tip 94 and a stiff proximal portion 93, and a docking handle 95. The docking handle houses a mechanism to tension at least one pull-wire coursing the length of the docking sheath 92 by turning the docking knob 96. The docking handle 95 also includes features to facilitate mounting of the docking system for stabilization, and a hub for the hemostasis valve and flush port 97. In some cases, the docking system 91 can be configured with a docking mounting unit 98 shown in Figure 6 on the proximal and distal ends to facilitate mounting the docking system to a delivery system platform.

In some cases, the docking sheath 92 can be configured with an inner diameter of 24Fr with a coil supporting structure within the wall to prevent kinking while bending. Further, the docking sheath 92 can be configured with a compliant distal tip 94 which is capable of bending a maximum of about 180 degrees, a stiff proximal portion 93, and at least one pull-wire traveling along the sheath length. In some cases, bending of the docking sheath distal tip 94 can be controlled by tensioning the pull-wire.

In some cases, the docking sheath 92 pull-wire can be attached to a docking handle 95. The docking handle 95 can be configured with a mechanism to tension the pull-wire in order to bend the distal tip 94 of the docking sheath 92. In some cases, the pull-wire can be attached to a pulley and set of gears controlled by a docking knob 96 within the docking handle 95, where turning the docking knob 96 in a first rotative direction can turn the gears and pulley causing the pull-wire to wrap around the pulley and tension the pull-wire, and turning the docking knob 96 in a second rotative direction back to its initial position can release tension from the pull-wire. By doing so, the bending of the docking sheath distal tip 94 can be precisely controlled.

In some cases, the docking sheath 92 pull-wire can be attached to a linear displacement mechanism within the docking handle 95 which can selectively tension and release tension on the pull-wire, such that bending of the docking sheath distal tip 94 can be precisely controlled.

One skilled in the art can appreciate that the docking sheath 92 can be configured to be steerable to facilitate navigation of the docking sheath 92 to the heart.

It is contemplated that the DGF members 61 can be delivered in one or more steps to the native mitral annulus 5 within the docking system 91 with a DDM 101.

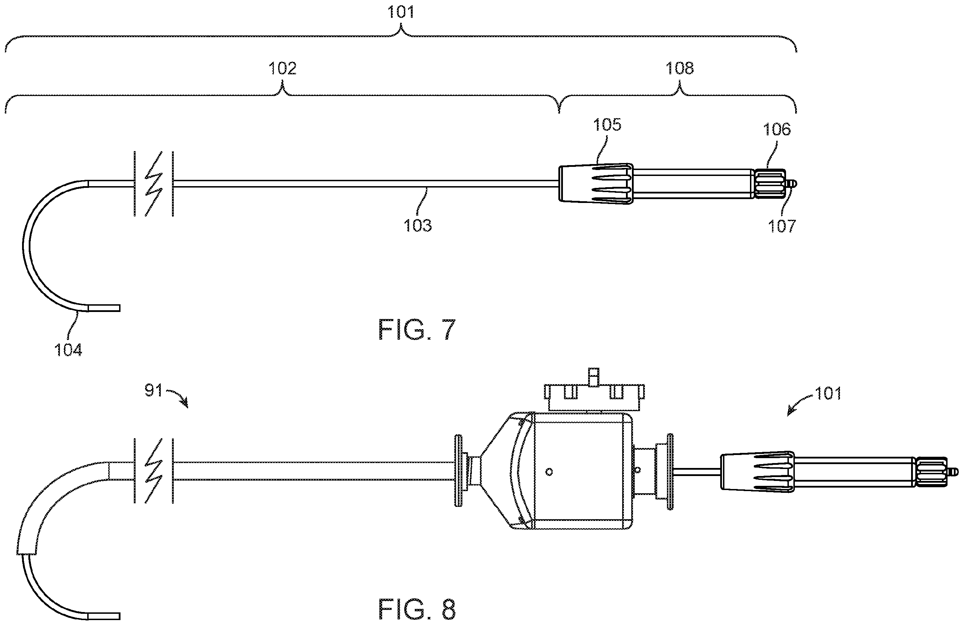

In some cases depicted in Figure 7 the DDM 101 can consist of a steerable outer sheath 102 with a control knob 105, a torque-driving shaft 111 with a control knob 106, and a DGF tail member grabber 107. The steerable outer sheath 102 is configured with a compliant distal section 104, a stiff proximal section 103, and at least one pull-wire along its length. The pull-wire at the proximal end of the steerable outer sheath 103 is linked to a controller with a tensioning mechanism which can be operated to bend the distal tip of the sheath to a maximum angle of 180 degrees. The torque-driving shaft 111 is configured to fit inside the steerable outer sheath 102, and to transmit torque from the proximal to distal ends, such that rotating the torque-driving shaft controller 106 can rotate the distal tip of the torque-driving shaft 112. A DGF tail member grabber 107 at the proximal end of the torque-driving shaft 111 is used to link the DGF member 61 to the DDM 101.

The DDM 101 can be configured to fit within the docking system 91. In operation, the docking sheath92 can be inserted into the left atrium 1 via transseptal delivery first. To implant the DGF members 61, the DDM 101 can be inserted into the docking system 91. In this configuration the docking system 91 and the DDM 101 offer two degrees of freedom for delivery and implantation of the DGF members 61.

In some cases, the torque-driving shaft 111 can be configured with a hollow lumen to accommodate the DGF tail member 70 passage to the proximal end of the MSMI, delivery system.

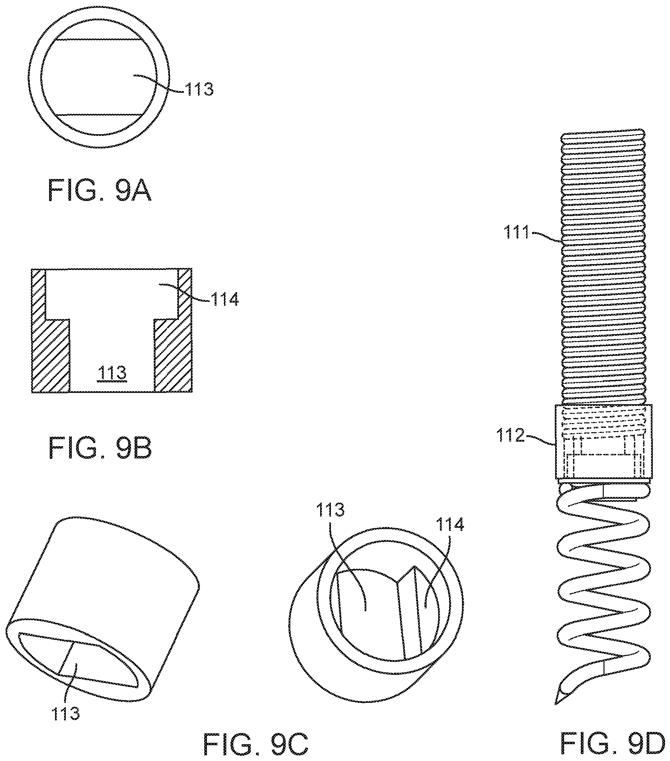

Referring to Figure 9, in some cases, the DDM torque-driving shaft 111 can be configured to engage a DGF engager 65. The distal tip 112 is configured with a slot 113 that is specially designed such that the protrusion on the distal portion of the DGF body member 64 can fit within the slot 113. Figure 9D is a perspective view of the torque-driving shaft 111 engaged with the DGF engager 65. The torque-driving shaft can have a hollow structure, such that the DGF member tail 70 can pass through it. The DGF tail member 70 is looped through the through hole on the DGF body member 66 and passed through the hollow lumen of the torque-driving shaft 111. The DGF member can be selectively held in place on the distal tip of the DDM torque-driving shaft 112 by tying or otherwise fixing the DGF member tail 70 at the proximal end of the DDM 101 with a DGF tail member grabber 107, and can be released by releasing the DGF tail member grabber 107.

In some cases, the torque-driving shaft 111 can be configured to fit inside the lumen of the steerable outer sheath 102. The steerable outer sheath 102 can be configured with a stiff proximal section 103, a compliant distal section 104, and a handle 108 on the proximal end to allow the operator to precisely control bending of the distal tip 104 of the steerable outer sheath 102. In some cases the steerable outer sheath 102 can be used to navigate to the DGF member 61 implantation site, and once in position, the torque-driving shaft 111 can be used to drive the DGF member 61 into the tissue.

In some cases, the DDM torque-driving shaft 111 can be configured as a hollow tube. In some cases, the torque-driving shaft 111 can be configured as a hollow coil, rope, or a series of twisted wires enabling torque transmission from the proximal to distal ends in bent configurations. The torque-driving shaft 111 can be configured to be highly flexible in bending such that it can bend with minimal force, and the steerable outer sheath handle 108 can be used to bend the torque-driving shaft 111 that is within the steerable outer sheath 102.

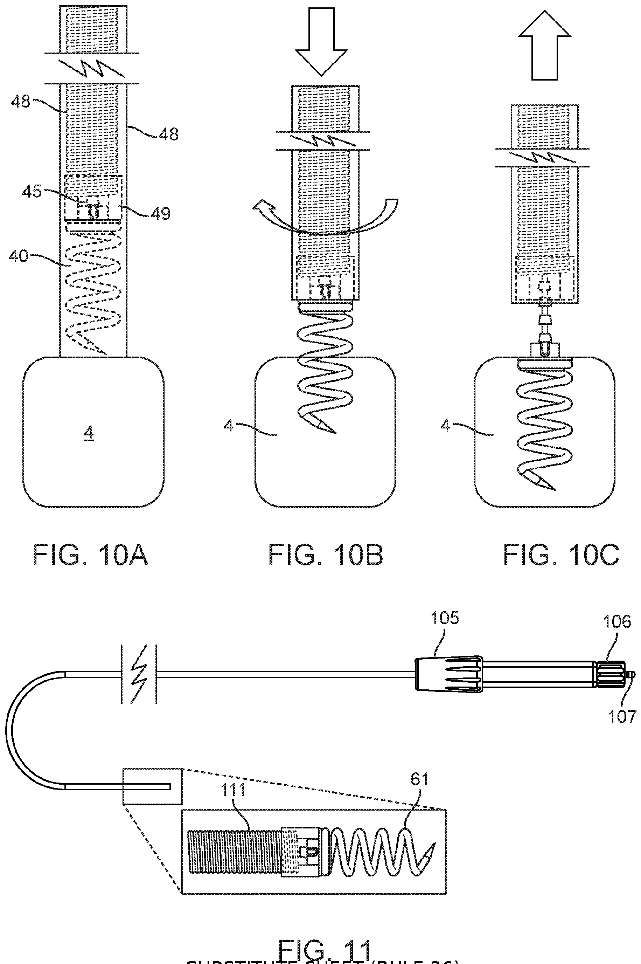

Referring to Figures 10A - 10C in order to deploy a DGF member 61 into the tissue, the DGF body member 64 is first engaged in the distal tip of the torque-driving shaft 1122, and the DGF tail member 70 is passed through the torque-driving shaft 111 and tied at the proximal end of the DDM with a DGF tail member grabber 107. The torque-driving shaft 111 is then inserted in the steerable outer sheath 102 and advanced towards the tissue. The torque-driving shaft 111 is turned in the first rotative direction to drive the DGF head member 62 into the tissue. Once the DGF head member 62 is implanted in the tissue, the DGF tail member grabber 107 is released to release the DGF tail member 70 to disengage the DGF body member 64 from the torque-driving shaft 111, and the DDM 101 is retracted from the body.

Figure 11 shows one example of the DDM 101 which is configured to implant a DGF member 61 into the native annulus 5. In some cases, the torque-driving shaft 111 is inserted into the outer steerable sheath 102. The torque-driving shaft 111 is designed to be highly flexible and to transmit torque from the proximal torque-driving shaft controller 106 to the distal tip 112 while it is being bent by the steerable outer sheath 102. One skilled in the art can appreciate that some cases of the DDM 111 enables the implantation of DGF members 61 in tight, difficult-to-navigate-to locations within the body.

One skilled in the art can appreciate that tangling of the trailing DGF tail members 70 following deployment of the DGF head members 62 to the native annulus 5 could lead to catastrophic events during prosthetic valve 34 delivery, including DGF member 61 pull-out and failure to lock the prosthetic valve 34 into position. Thus, in some cases, the DGF tail members 70 trailing outside the body, can be organized and separated with an expandable compartmentalization (EC) sheath 121. In some cases, the DGF tail members 70 are passed through the Expandable Compartmentalization (EC) sheath 121 which is configured with multiple lumens. The EC sheath lumens can be configured to allow for passage of the DDM 101 and to separate and compartmentalize each of the DGF member tails 70. The EC sheath 121 can be inserted into the docking sheath 91 during DGF member 61 deployments.

In some cases, the EC sheath 121 is a tubular structure with one or more hollow lumens, depending on the number of DGF members 61 to be implanted, typically a minimum of three lumens. Once a DGF member 61 is implanted along the annulus 5 or sub-annulus, the DGF tail member 70 can exit the body and come out the proximal end of the docking sheath 91. Each of the DGF tail members 70 can then be identified and separated using the EC sheath 121 to prevent tangling of the DGF tail members 70.

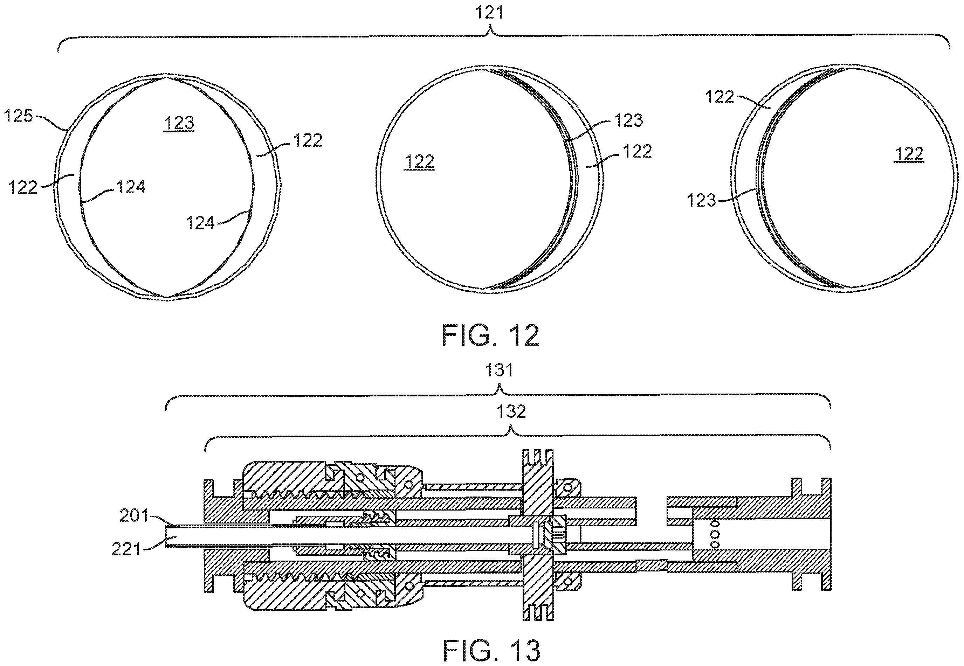

Figure 12 depicts one example of the EC sheath 121 wherein the EC sheath 121 is configured as a tube with a flexible inner wall 124 which can be collapsed and pushed to either side. The EC sheath 121 can be used to organize DGF tail members 70 between multiple DGF member 61 deployments in order to prevent tangling or twisting of the DGF tail members 70. One skilled in the art can appreciate that the nature of the collapsible lumens provides a mechanism to maximize the diameter of the lumen being actively used, while minimizing the overall diameter of the EC sheath 121. It is important to note that reducing the overall diameter of the MSMI, delivery system is important for patient safety.

In some cases, following the implantation of the DGF members 61, the prosthetic valve 34 and plurality of DGF locking members 81 are delivered and implanted in native mitral valve.

The MSML delivery system can be configured with a valve delivery system 131 distinct from the DDM 101. The valve delivery system 131 can be configured to deploy the prosthetic valve 34 and the additional DGF locking members 81 on top of the prosthetic valve 34.

In some cases, the heart valve leaflet replacement device 47 can be delivered to the native mitral annulus 5 from a trans-atrial or trans-septal approach via catheter. The prosthetic valve 34 can be guided to the precise position by a plurality of DGF members 61 configured so that the DGF member tail 70 passes through the stent 32 and attaches to the head portion of the DGF members 62 previously embedded in the native annulus 5 via a transcatheter approach. Once in the operative position, the prosthetic valve 34 can be locked in place against the annulus 5 at the deployed DGF member bodies 64 by releasing a plurality of DGF locking members 81 on top of the atrial flared portion 41 of the stent 32 with the MSML delivery system.

In some cases, the tail members 70 of the previously implanted DGF members 61 can be configured to pass through specially designed holes on the atrial upper flared portion of the stent 44. Alternatively, the DGF tail members 70 can pass through holes in the skirt material of the prosthetic valve 34.

The following description outlines at least one method in which the valve deployment handle can be assembled, as depicted in Figure 13. One skilled in the art can appreciate that the valve deployment handle 131 can be arranged in many different ways depending on the order in which each function is to occur. A distal mounting unit 152 is secured at the distal end of the sleeve 151, which can be used for mounting onto a stabilization platform. The sleeve driver 171 can sit at the distal end of the sleeve 151 on the sleeve helix 158. Proximal to the sleeve driver 171 is the hemostasis-tubing housing compartment 181. The valve sheath compartment 205 can be inserted into the interior of the sleeve 151 with a flush port 208 that sticks out of the hemostasis-tubing housing compartment 181. The LHS locking compartment 222 can sit proximal to the hemostasis-tubing housing compartment 181. The LHS compartment 181 can be inserted into the interior of the sleeve 151, proximal to the valve sheath compartment 205, and can be secured within the LHS locking compartment slit 192 via the LHS compartment shell levers 228. The stabilization appendages 224 on the LHS compartment 222 can align with the sleeve ridge 161. The LHS compartment shell 226 can lock the LHS compartment 222 into place via the radial slits 160 on the sleeve 151. A proximal mounting unit 156 is secured at the proximal end of the sleeve 151, which can be used for mounting onto a stabilization platform.

In some cases, the valve delivery system 131 can be configured such that the main valve sheath 201 housing the crimped stent 141 goes through the docking sheath 92. When the prosthetic valve 34 is crimped into a smaller size, it is loaded into the distal end of the valve sheath 202 and is adjacent to the distal end of the LHS system 244. A plurality of the DGF locking members 81 and DGF locking member sheaths 251 can be loaded into the distal end of the LHS system 244, which can allow the DGF locking members 61 to be deployed immediately after the prosthetic valve 34 is released.

The following description details at least one method for prosthetic valve 34 delivery using the valve delivery system 131. The technique described in this paragraph is dependent on one instance of assembly of the valve delivery handle 132. Before the valve delivery handle 132 can deliver a prosthetic valve 34 into the desired location in the body, the DGF locking member 81 and prosthetic valve 34 must be crimped into the distal end of the valve sheath 202. The valve delivery handle 131 can be mounted onto the delivery platform and placed at the distal end of the docking sheath 94 into the right atrium 1. Retracting the sleeve driver 171 can in turn retract the hemostasis-tubing housing compartment 181, the lock housing structure locking compartment 191, and the valve sheath compartment 205 simultaneously. The lock housing structure compartment 222 is locked in place via the radial slit 160 in the sleeve 151. The sleeve driver 91 can continue to be retracted until it is off the sleeve helix 158. This signifies that the prosthetic valve 34 is completely released from the valve sheath 201 and the valve sheath compartment 205 is fully retracted to the lock housing structure compartment 222. To retract the valve sheath 201 out of the right atrium 1, the LHS compartment knob 229 can be rotated in the unlock position at the distal slit radial of the sleeve 160. The plurality of compartments can be fully retracted to the proximal end of the sleeve 151, and the LHS compartment knob 229 can be rotated into the lock position in the proximal radial slit of the sleeve 160. This allows passage for the DGF locking member sheaths 251 to be advanced to begin the process of locking the prosthetic valve 34 in place.

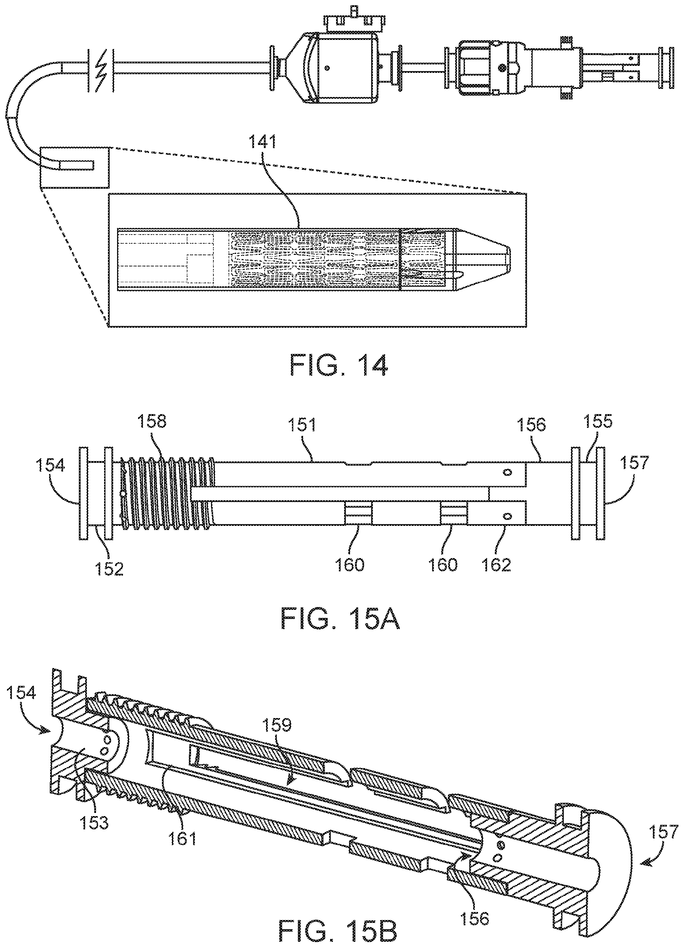

Figure 14 shows one example of valve delivery system 131 inside the docking system 91 configured to implant the prosthetic valve 34 in the native annulus 5 at the location of the previously implanted DGF members 61. The crimped valve stent 141 is loaded into the distal tip of the valve sheath 202. The valve sheath cap 271 covers the distal tip of the ventricular portion of the prosthetic valve 42 in continuity with the valve sheath 201. The DGF locking member sheath 251 and distal portion of the LHS 244 are positioned just proximally to the crimped valve stent 141. In operation, the valve sheath 201 is advanced within the docking sheath 92 and the docking sheath 92 is bent from the atrial septum 11 to the native mitral annulus 5 by turning the docking knob 95, to direct the valve sheath 201 towards the left ventricle 2 for prosthetic valve deployment 34.

In some cases of the valve delivery system 131, the sleeve 151 consists of the sleeve helix 158 and distal 152 and proximal 156 mounting units. Both the valve sheath 201 and the LHS sheath 221 are fixed to their respective compartments within the sleeve 151, which controls the movements of the sheaths along the sleeve 151. During prosthetic valve 34 release, the LHS sheath 221 remains stationary proximal to the crimped stent 141 within the valve sheath 201. The valve sheath 201 is linked to the sleeve driver 171 which can be moved along the sleeve helix 158. The prosthetic valve 34 can be thereby released from the valve sheath 201 by rotating the sleeve driver 171. In some cases, the sleeve 151 can be mounted horizontally or at an angle on the platform using the mounting units attached to the distal 153 and proximal ends of the sleeve 156.

Figure 15 depicts one example of the valve delivery system handle sleeve 151. In some cases, the sleeve 151 consists of distal 154 and proximal portions 157, and distal 152 and proximal 156 mounting units. The distal portion 154 is configured with a helix 158 which engages the helix on the sleeve driver 172 which is configured to link with the hemostasis-tubing housing compartment 181, housing the valve sheath compartment 205, such that turning the driver 171 can change the position of the valve sheath 201 along the sleeve 151. The proximal portion 157 is configured with a longitudinal slit 159 and two radial slits 160. The longitudinal slit 159 provides a mechanism to move the valve sheath 201 and LSH sheath 221 along the sleeve 151, while the radial slits 160 provide a mechanism to lock the position of the valve 201 and LHS sheath 221 along the sleeve 151. The sleeve 151 is configured with a distal 153 and proximal 156 mounting units on either end which are used to aid in stabilization of the valve delivery system 131.

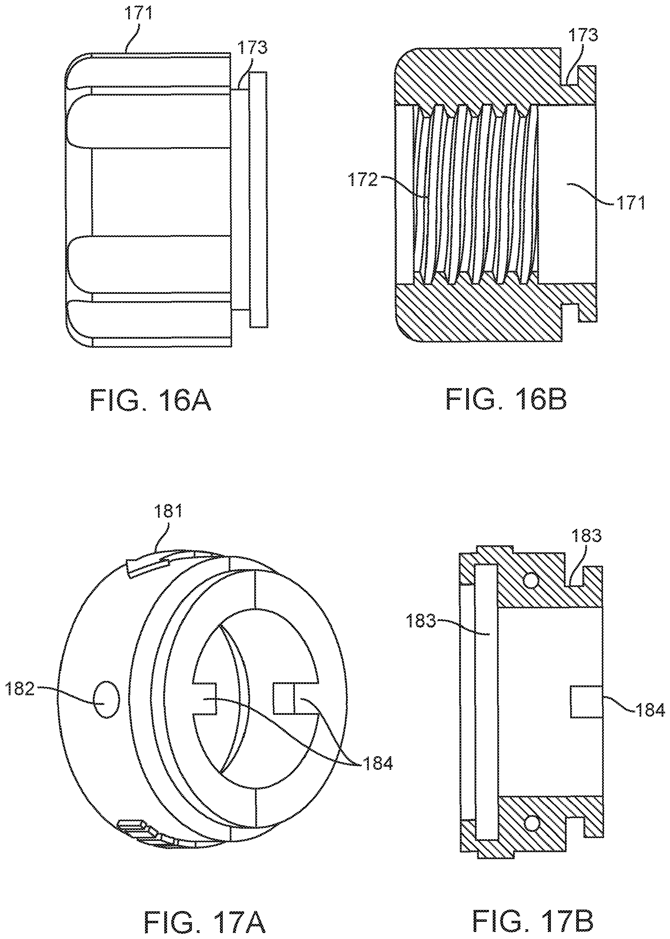

Figure 16 depicts one example of the valve delivery system sleeve driver 171. In some cases, the sleeve driver 171 is configured with ergonomic grips on the outside, a helix 172 on the inside which engages with the helix on the sleeve 158, and an attachment mechanism 173 to engage with the hemostasis-tubing housing compartment 181.

One skilled in the art can appreciate that many different sleeve 151 and sleeve driver 171 designs can be used to control the relative linear displacement of the valve 201 and LHS 221 sheaths within the valve delivery system handle 132.

In some cases, the hemostasis-tubing housing compartment 181 of the valve delivery system 131 is configured with an opening 182 for insertion of a flush port 208 from the valve sheath compartment 205, static appendages 184 designed to engage the sleeve 151, and distal and proximal attachment mechanisms 173 to engage with the sleeve driver 171 and LHS locking compartment 191 as illustrated in Figure 17.

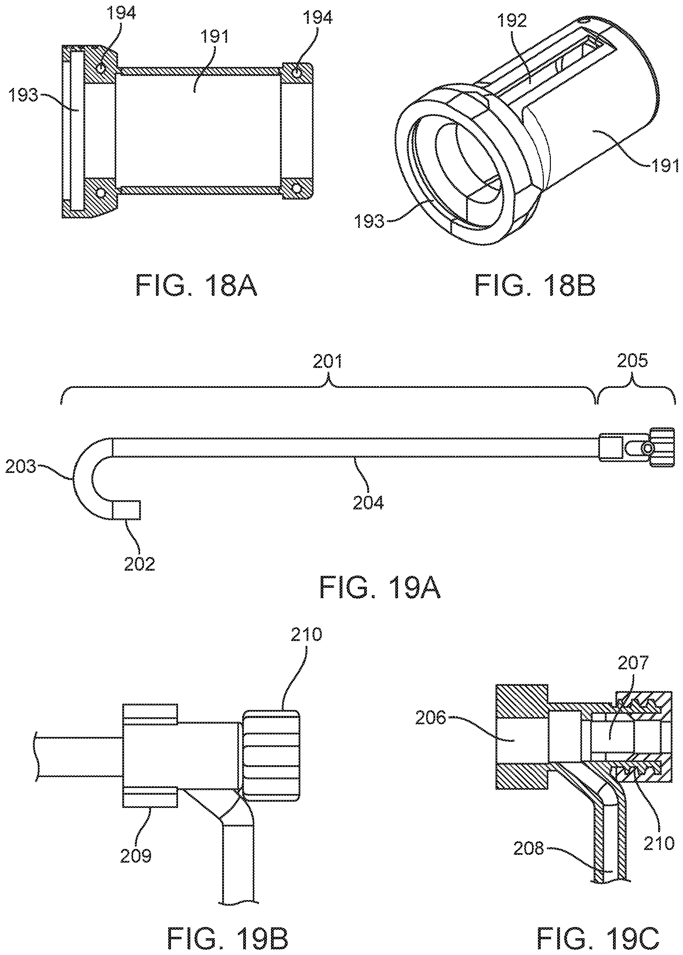

One example of the LHS locking compartment 191 is depicted in Figure 18. In some cases, the LHS locking compartment 191 is configured with an attachment mechanism 193 to engage with the hemostasis-tubing housing compartment 181, threaded holes 194 to facilitate attachment of the two halves of the compartment 191, a barrel to allow passage over the sleeve 151, and two longitudinal slits 192 used as channels to allow the levers 228 of the LSH compartment shell 226 to engage with the radial slit of the sleeve 160.

Figure 19 depicts one example of the valve sheath 201. In some cases, the valve sheath 201 consists of three sections: a distal section 202, a bending section 203, and proximal section 204. The distal section 202 is where the crimped prosthetic valve 141 sits within the valve delivery system 131. The distal valve sheath 202 section is stiff to resist deformation due to radial and axial forces induced by the crimped stent 141. The bending section 203 is compliant such that the valve sheath tip 202 can make a maximum bend angle of 180 degrees. The proximal portion 204 of the valve sheath 201 is stiff to provide pushability.

The valve sheath 201 is linked to the valve delivery system handle 132 via the valve sheath compartment 205. In some cases, as shown in Figure 19, the valve sheath compartment 205 is configured with proximal and distal 206 portions. The distal portion of the valve sheath compartment 206 is configured with a locking appendage 209 that fits into ridges 161 in the sleeve 151. The proximal end of the valve sheath 204 is attached to the distal portion of the valve sheath compartment 206. A flush port 208 sits proximal to the valve sheath 201 and exits the opening on the hemostasis-tubing housing compartment 181, a hemostasis valve 207 sits proximal to the flush port 208. The proximal portion of the valve sheath compartment 206 is configured as a cap 210 with a helix that corresponds to the helix on the distal valve sheath compartment 206, such that it can be screwed on to prevent leaking.

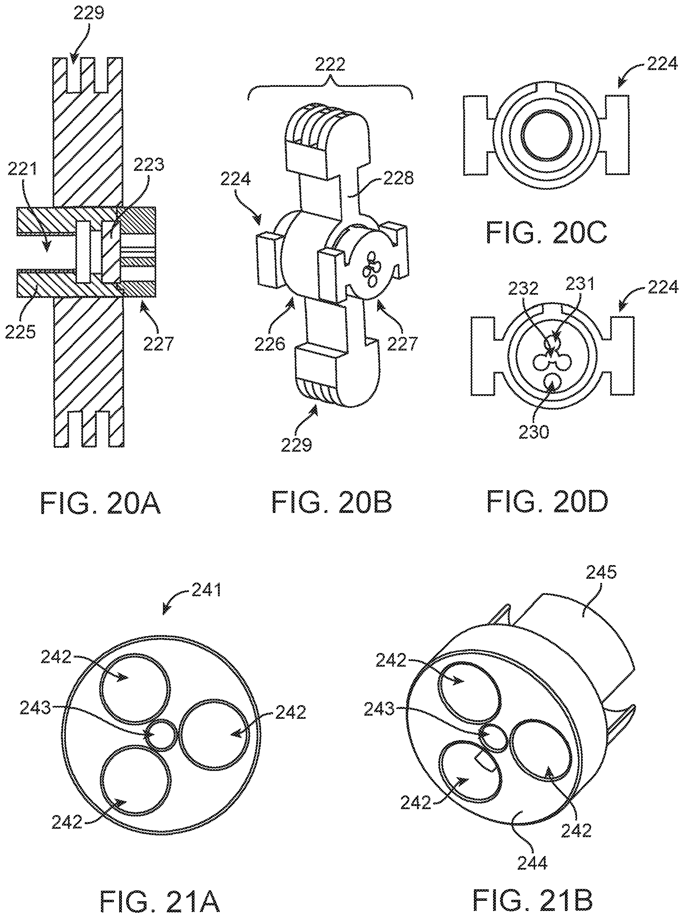

In some cases of the LHS compartment 222 shown in Figure 20, the LHS compartment 222 consists of the LHS compartment body 225, shell 226, and cap 227. The LHS sheath 221 is attached to the distal side of the LHS compartment body 225, a LHS hemostasis valve 223 sits proximal to the LHS sheath 221. The LHS compartment shell 226 is configured to turn around the stationary LHS compartment body 225. The LHS compartment shell lever 228 is configured to fit within the longitudinal slits 192 in the LHS locking compartment 191 as well as the radial slits 160 in the sleeve 151. The knobs 229 on either end of the LHS compartment shell lever 228 are configured to allow a mechanism to easily lock the LHS compartment 222 position along the sleeve 151 by turning the lever 228 to engage the radial slits 160 in the sleeve 151. The LHS compartment cap 227 fits into the proximal side of the LHS compartment body 225. The LHS compartment cap 227 is configured with three lumens 231 for the locking member sheaths 251, one central lumen 232 for the valve stabilization mechanism tube 278, and one lumen 230 to attach a flush tube. Both the LHS compartment body 225 and LHS compartment cap 227 are configured with stabilization appendages 224 on either side that fit in ridges 160 on the sleeve 151.

A LHS 241, can be configured to operatively house the DGF tail members 70, the DGF locking members 81, and the DGF locking member sheaths 251. In some cases, the LHS 241 can define a proximal portion 253 and a distal portion 252, where the distal portion 252 contains a plurality of lumens for the DGF tail members to pass through. Optionally, only a portion of the distal tip of the LHS 241 can be configured to define the plurality of lumens for the DGF tail members 70 to pass through. In some cases, the dimensions of these lumens are configured to house a plurality of DGF locking members 81 and the corresponding DGF locking member sheaths 251. It is contemplated that with a plurality of DGF locking members 81 stored in the LHS 241, a plurality of DGF locking members 81 could be delivered simultaneously upon the release of the prosthetic valve 34.

An example of the LHS 241 is shown in Figure 21. In some cases, the LHS 241 has distal 244 and proximal 245 portions. The proximal portion 245 fits inside the lumen of the LHS sheath 221, the distal portion 244 fits outside the LHS sheath 221. There are four lumens: three lumens to house DGF locking members and locking sheaths 242, and a central lumen for the valve stabilization mechanism 243.

Figure 22 shows an example of the DGF locking member sheaths 251. The distal portion of the locking sheath 252 is configured with a locking member holder 254 which secures the DGF locking member 81 in the LHS 241 until it is deployed over the DGF locking units 81. The DGF locking member sheaths 251 consists of proximal 253 and distal 252 sections made of heterogeneous materials. The proximal section 253 can be made of rigid materials such as stainless steel, Nitinol and metals of the like. The stiffness and rigidity of the proximal section of the DGF locking member sheaths 251 is important for the purpose of pushing and releasing the locks. Whereas the distal section 252 of the DGF locking member sheaths 251 can be made of flexible and semi-rigid materials, which allow the section to bend during the valve deployment. The wire reinforced catheter, either coil or braided wire, can be used for such properties. The locking member holder 254 can be made of flexible, semi-rigid or rigid material, such as plastics, resins, silicon, metals, or the like. The locking member holder 254 has a proximal portion to facilitate attachment to the locking member sheath 251, a central portion to house the DGF locking member 81, and a distal portion 244 with radial protrusions that can keep the DGF locking member 81 inside the locking member holder 254 until the locking sheath 251 is advanced over the DGF locking units 67 which can expand the radial protrusions outward such that the locking member 81 can be deployed. This example of the LHS 241 is configured to house three DGF locking members 81 and locking sheaths 251 simultaneously.

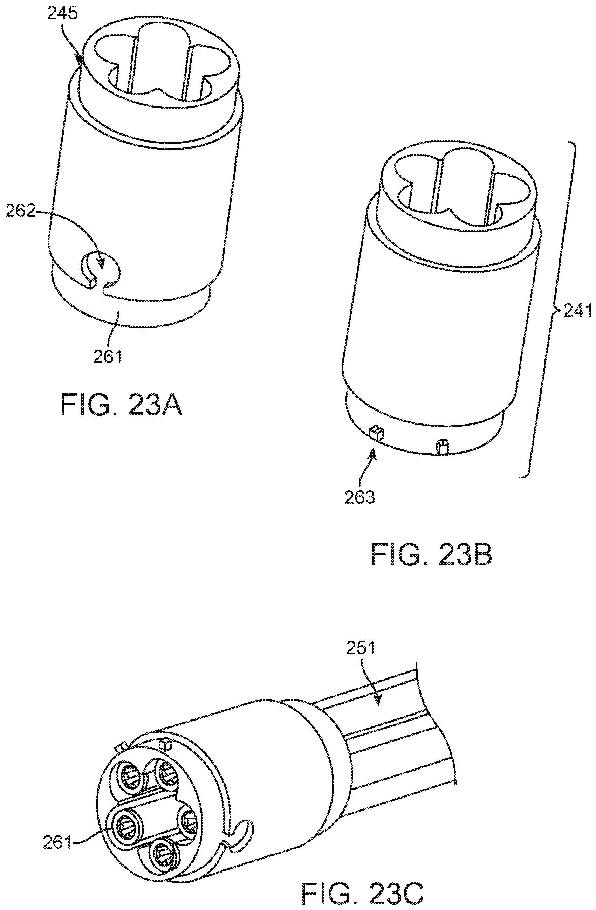

An alternative example of the LHS 241 which is configured with five outer lumens to house five DGF locking members 81 and locking sheaths 251 in Figure 23. In some cases, a central lumen 243 to house a valve stabilization mechanism 278, guidewire, or other catheter. Further in some cases, five DGF locking members 81 could be delivered simultaneously.

Optionally, the LHS 241 can be configured to also include a mechanism to aid in loading the prosthetic valve 34 into the valve delivery sheath 201. In one example shown in Figure 23A, the LHS 241 can be configured with a proximal recess to facilitate attachment of the LHS 241 to a LHS sheath 221, a distal cylindrical recess 261 which allows the proximal portion of the crimped valve stent 141 to lie flat against the LHS 241, and distal eyelets 262 which allow the stent tabs to lie flat against the LHS. In some cases, the LHS can be configured with small distal protrusions 263 which fit within the crimped struts of the valve stent 141 as shown in Figure 23B.

One skilled in the art can appreciate that LHS recesses 261 and protrusions 263 illustrated in Figure 23, can be used to facilitate loading of the crimped prosthetic valve 141 into the valve delivery sheath 201. In some cases, the prosthetic valve 34 can be crimped onto the LHS 241 such that the stent tabs 45 and struts become engaged with the corresponding LHS recesses 261 and protrusions 263 in the fully crimped configuration 141. The LHS sheath 221 can then be retracted into the valve delivery system handle 132 to drag the crimped prosthetic valve 141 into the valve delivery sheath 201.

In operation, a plurality of DGF locking members 81 are housed within the LHS sheath 221, which can sit proximal to the crimped stent 141 when loaded in the valve sheath 201. The position of the DGF locking members 81 within the LHS 244 can be controlled with a plurality of locking member sheaths 251 each connected to a control stud 295 in the DGF locking sheath chamber 291. In some cases, the DGF locking sheath chamber 291 can be configured with a plurality of DGF tail member control studs 295 which can be used to tension the DGF tail members 70.

In one optional embodiment, the LHS system 222 is coupled to the sleeve 151 to prevent rotation within the handle. Additionally, the valve sheath 201 can be configured such that it can not rotate as it is advanced towards the targeted implant location within the native mitral valve, for example, during delivery. One advantage of this non-rotational feature is that it prevents the DGF tail members 70 from being tangled within the valve delivery system 131.

In some cases, it is contemplated that the prosthetic valve 34 can be delivered by retracting the valve delivery sheath 201 over the crimped prosthetic valve 141 while keeping the LHS 241 stationary. In some cases, it is contemplated that there can be a mechanism to selectively prevent the LHS 241 from moving during prosthetic valve 34 deployment.

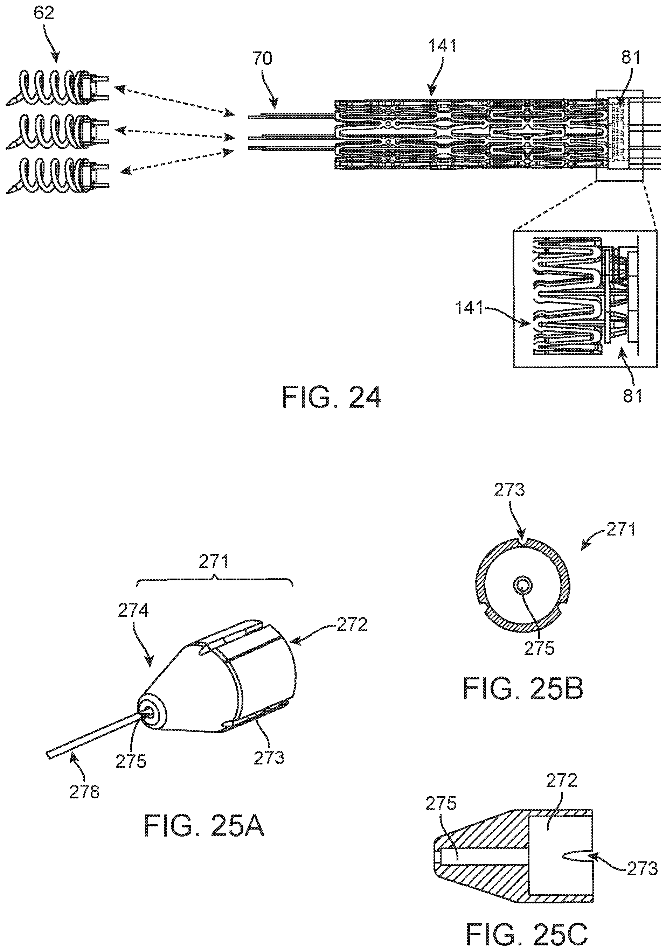

The valve delivery system 131 is linked to the previously deployed DGF members 61 via the DGF tail members 70 as shown in Figure 24. In some cases, the DGF tail members 70 trailing from the previously implanted DGF head members 62 are threaded through the crimped valve stent 141 through the openings on the atrial flare portion of stent 44 within the distal portion of the valve sheath 202, and then through the corresponding DGF locking members 81 in the LHS 241 proximal to the crimped prosthetic valve 141, and out the proximal end of the valve delivery system handle132.

In operation, the DGF tail members 70 threaded through the valve delivery system 131 act as rails to guide the delivery and implantation of the prosthetic valve 34 and DGF locking members 81.

In some cases, a valve stabilization mechanism 276 linking the valve stent 34 to the MSML delivery system can be used to keep the valve 34 in position at the annulus 5 following release from the valve sheath 201 such that rapid pacing is not needed during valve release and lock deployment. The valve stabilization mechanism 276 can then be removed after deployment of the locks 81. One skilled in the art can appreciate that in some cases, rapid ventricular pacing may not be necessary, and/or the operator may have more time to deliver the locking members to secure the valve stent in place at the annulus 5.

It is contemplated that the valve stabilization mechanism 276 can be configured as a looped suture 277 and a tube 278, where the suture 277 is looped through portions of the valve stent 32, with the two free ends of the suture 277 exiting through the tube 278 and out of the proximal end of the MSMI, delivery system. The two ends of suture 277 can be selectively tied to link the prosthetic valve 34 to the MSML delivery system, and then the suture 277 can be easily removed from the body by pulling one end from the proximal end of the valve delivery system 131.

In an optional embodiment, the valve delivery system131 can also comprise a valve sheath cap 271 which is placed over the distal end of the crimped stent 141. One skilled in the art can appreciate that it is possible for the DGF tail members 70 to get caught on the valve stent 32 during valve loading and/or release. It is contemplated that the valve sheath cap 271 can be used to cover the ventricular struts 42 on the valve stent to prevent the DGF tail members 70 from getting caught under the stent during valve release. Following valve release, the valve sheath cap 271 can be advanced to fully release the prosthetic valve 34, and then retracted back into the MSML delivery system and removed from the body.

In an optional embodiment, the valve stabilization mechanism 276 can comprise a tube 278 to house the suture 277 linking the prosthetic valve 34 to the MSML delivery system which is configured to attach to a valve sheath cap 271. The valve sheath cap 271 can be configured to cover the lower stent struts on the ventricular portion 42 of the crimped valve stent, such that the DGF member tails 70 do not get caught on the lower stent struts of the valve stent 42 during valve deployment. The valve stabilization mechanism tube 278 can be advanced to push the valve sheath cap 271 towards the ventricle 2 and fully release the prosthetic valve 34 once the valve sheath 201 has been retracted. Once the locks 81 have been deployed over the atrial flared portion of the valve 41, the valve sheath cap 271 can be retracted by retracting the valve stabilization mechanism tube 277 back into the docking sheath 92.

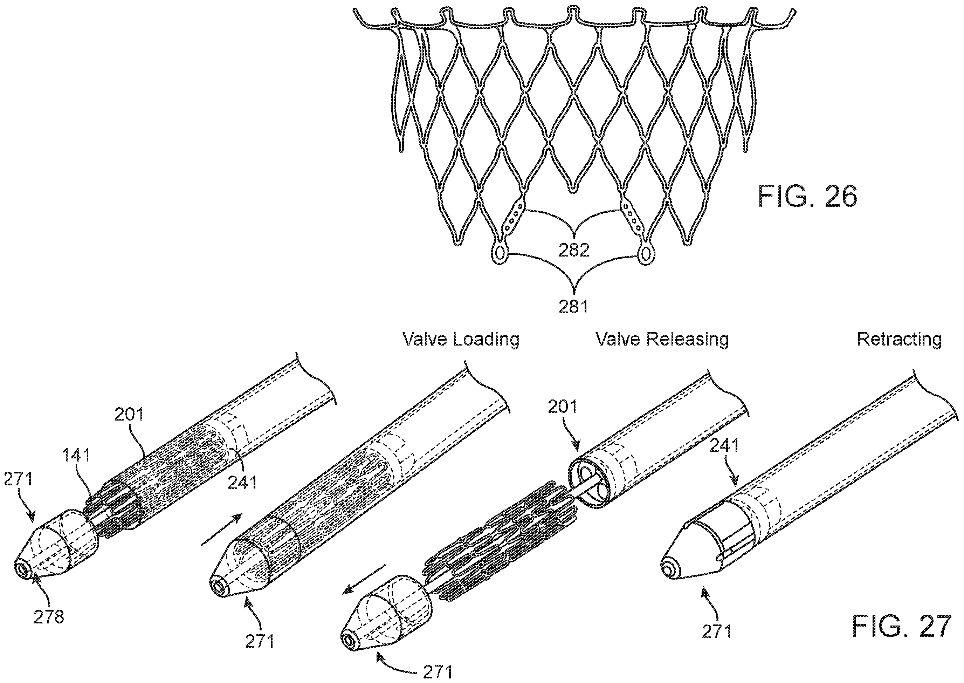

One example of the valve sheath cap 271 is shown in Figure 25. In some cases, the valve sheath 201 has a distal portion 202 that is configured to fit over the proximal portion of the crimped prosthetic valve 141, a central lumen which provides a means for the valve stabilization mechanism tube 278 or passage of a guidewire or other catheter, and a distal tip 274 which is rounded or conical in shape to aid in the navigation of the valve sheath 201 within the body. The valve sheath cap 271 can be configured with longitudinal slots 275 to organize the DGF tail members 70, which in operation would trail from the DGF member bodies 64 previously implanted at the native annulus 5 through the valve 131 and lock 291 delivery systems. Further in some cases, the valve stabilization mechanism 276 can be configured as a suture 277 which is looped through the openings on the lower ventricular portion of the valve stent shown in Figure 26 and then around the outside of the valve sheath cap and into the valve 281 stabilization mechanism tube 278 running through the center of the valve sheath cap 271.

In some cases, the proximal hollow portion of the valve sheath cap 272 can be configured with a series of collapsible teeth in the shape of a hollow cone pointing proximally which can be opened into a cylindrical shape in order to sheath a portion of the crimped prosthetic valve 141, and can collapse once the crimped prosthetic valve 141 is released from the valve sheath 201 back to its conical shape. One skilled in the art can appreciate that the conical shape of the valve sheath cap 271 can facilitate retraction of the valve sheath cap 271 back into the MSMI, delivery system following valve deployment.

In operation, the valve sheath cap 271 is pushed over the distal tip of the crimped ventricular portion 42 of the valve stent 32 such that it is in continuity with the valve sheath 201 when loading the prosthetic valve 34 into the valve delivery system 131. During valve release, the valve sheath cap 271 can be advanced by the valve delivery handle 131 to release the distal ventricular portion of the portion 42 of the crimped valve stent 141. Following release of the valve 34, the valve sheath cap 271 is retracted back towards the valve sheath 201, through the docking sheath 92, and removed from the body. In some cases, the valve sheath cap 271 can be configured to attach to the valve stabilization mechanism tube 278 which has a controller on the proximal end of the valve delivery system 131, wherein adjusting the position of the valve stabilization mechanism tube controller can in turn adjust the position of the valve sheath cap 271.

In some cases the DGF locking member sheaths 251 can be controlled by individual controls within the valve delivery system handle 131 housed in the DGF locking sheath chamber 291.

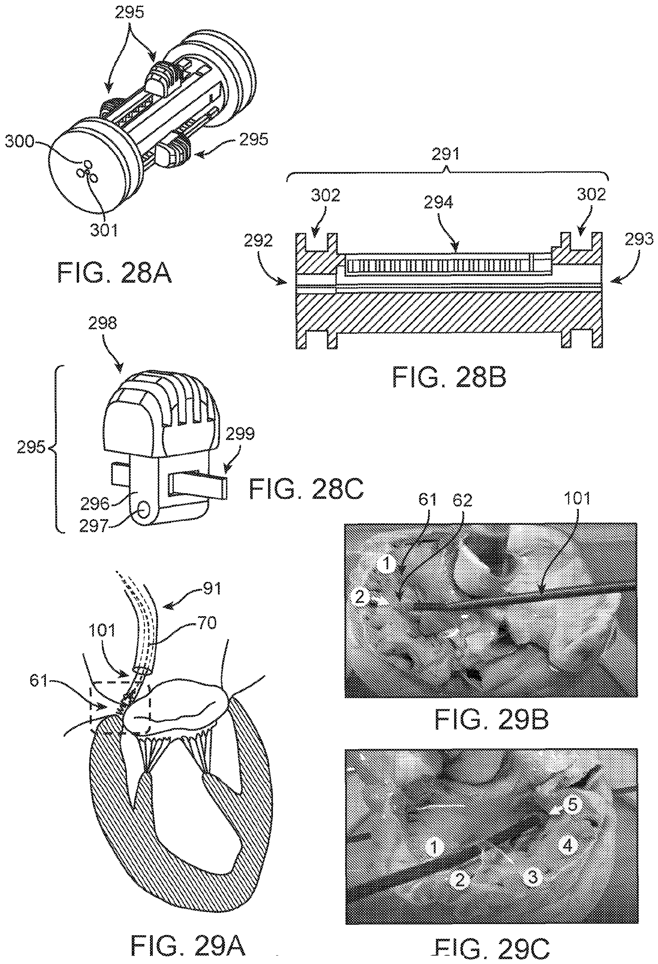

In some cases of the DGF locking sheath chamber 291 depicted in Figure 28, the DGF locking sheath chamber 291 can consist of mounting units 302 on the inlet 292 and outlet 293, and DGF locking sheath chamber sliders 294. In some cases, the DGF locking sheath chamber 291 has three DGF tail member lumens 300 and sliders 294 to accommodate 3 DGF locking member sheaths 251 and one central lumen 301 to accommodate passage of a valve stabilization mechanism tube 278, guidewire, or other catheter. The DGF locking sheath chamber stud body 296 fits into the DGF tail member lumen 300 and can travel along the DGF locking sheath chamber sliders 294. The stud 295 is configured to link to the DGF locking sheaths 251 through the body channel 297, such that pushing the stud 295 along the slider 294 can adjust the position of the DGF locking sheath 251. The DGF tail member 70 passes through the study body channel 297 to the proximal end of the valve delivery system in operation. The stud lever 299 engages ridges along the DGF locking chamber slider 294 such that the position of the stud 295 along the slider 294 can be precisely controlled and held in place. The stud has an ergonomic shaped head 298 for easy operation.

Retracting the valve sheath 201 can release the prosthetic valve 34. In some cases, the release of the distal end of the prosthetic valve 34 can be achieved below the annulus 5. When the distal end of the prosthetic valve 34 is partially released, the entire valve sheath 201 can be positioned across the valve annulus 5. The DGF locking member sheaths 251 can be advanced along the DGF member tails 70 immediately following the release of the prosthetic valve 34 to guide the prosthetic valve 34 into position at the deployed DGF members 61 and release the DGF lock members 81 to effectively lock the prosthetic valve 34 in the operative position.

It is contemplated that the plurality of DGF locking members 81 can be configured to be deployed from the valve delivery system 131 immediately following deployment of the prosthetic valve 34. One skilled in the art can appreciate that in some cases, the DGF locking members 81 can secure the prosthetic valve 34 after its release from the valve sheath 201 quickly within 30 seconds of rapid-pacing time.

It is contemplated that the plurality of DGF locking member sheaths 251 can be configured to selectively couple and decouple from the plurality of locking members 81, such that the locking members 81 can be left in the body and the locking member sheaths 251 can be removed from the body at the completion of the implantation procedure. In some cases, the locking member 81 may contain a plurality of L-slots that is configured to fit a plurality of pins at the distal tip of the locking member sheath 251, such that the locking member can be coupled to the locking member sheath 251 by rotating it in the first rotative direction. The locking member sheath 251 can then be used to deploy the locking member 81 over the locking units 67 on the DGF body member 64, before being selectively decoupled from the locking member 81 by rotating the locking member sheath 251 in the second rotative direction, opposing the first rotative direction. In some cases, the locking member 81 can be deployed and the locking member sheath 251 can be pulled proximally to test whether the locking member 81 is engaged with the locking units 67 before selectively decoupling the locking member 81 from the locking member sheath 251. One skilled in the art can appreciate that in some cases, the user can ensure that the heart valve leaflet replacement system 47 is securely and optimally locked down against the implanted DGF member heads 62 before removing the valve delivery system 131 and completing the implantation procedure.

Referring to Figure 28, the DGF locking sheath chamber 291 can be configured with a distinct DGF locking sheath stud 295 for each of the locking member sheaths 251, such that one or more locking members 81 can be deployed by operating the corresponding DGF locking sheath studs 295. Then each locking member 81 can be individually adjusted using one DGF locking sheath stud 295 at a time. One familiar in the art can appreciate that this can help ensure each locking member 81 is secure and in the optimal position before the valve delivery system 131 is removed from the body, which can reduce the risk of valve dislodgement and paravalvular leakage.

In some cases, the MSMI, system can be mounted on a platform proximal to the docking system 91 to stabilize the system during valve delivery.

In some cases, the heart valve leaflet replacement system 47 implantation procedure using the MSMI, delivery system is described in the proceeding sections, and illustrated in Figures 29 - Figure 35.

In some cases, a plurality of DGF members 61 can be sequentially implanted in the native mitral annulus 5. Subsequently, the prosthetic valve 34 can be delivered and positioned such that the atrial flared portion 41 of the stent 32 can be in close proximity to the implanted DGF head members 62. Optionally, it is contemplated that a method of retrieving the prosthetic valve 34 back into the valve delivery system 131 can be implemented to ensure the optimal delivery and positioning of the prosthetic valve 34 inside the native mitral annulus 5.

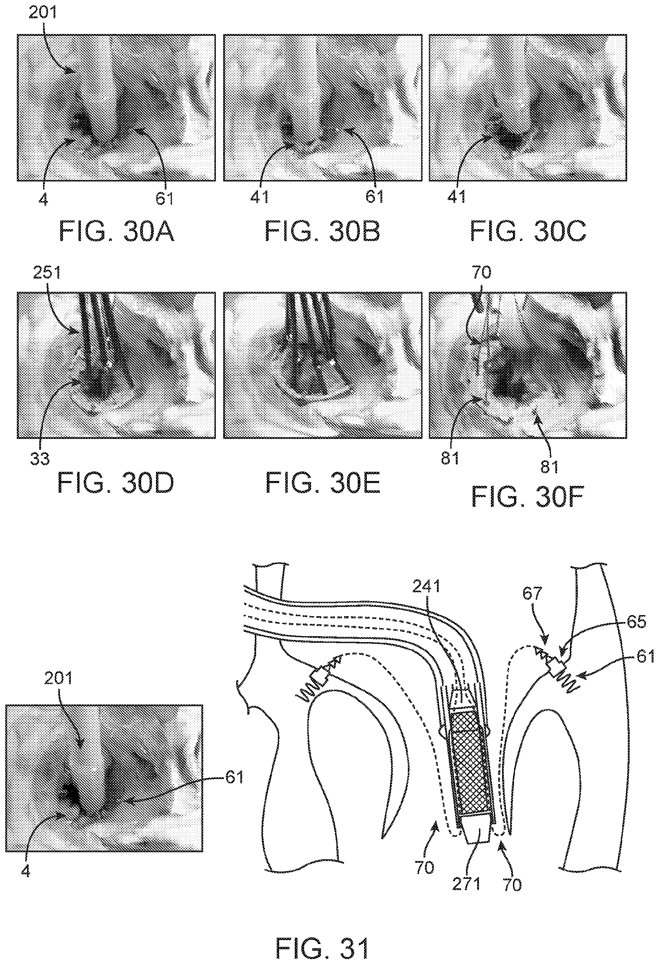

First DGF members 61 are deployed along the posterior mitral annulus 5 with the DDM 101 as shown in Figure 29. Then the valve sheath 201 is positioned for valve deployment within the annulus 5 as shown in Figure 30A. The valve sheath 201 is then incrementally retracted proximally into the valve delivery system handle 132 to release the prosthetic valve 34 as shown in Figures 30B and 30C. Once the prosthetic valve 34 is completely out of the valve sheath 201, the locking member sheaths 251 are advanced to the atrial flared portion 41 of the stent to deploy the DGF locking members 81 over the DGF locking units 67 as shown in Figures 30D and 30E. Once the locking members 81 have been deployed, the DGF locking member sheaths 251 are retracted back into the valve delivery system handle 132, and the DGF tail members 70 are sequentially removed leaving only the heart valve leaflet replacement system 47 in the native mitral valve. Figure 30F shows the valve delivery process after one DGF tail member 70 has been removed.

During the prosthetic valve 34 and DGF locking member 81 deployment procedure, the DGF tail members 70 can be slightly tensioned to guide the respective DGF locking member sheaths 251 as they are advanced towards the prosthetic valve 34 to push the prosthetic valve 34 towards the implanted DGF head members 62 and deploy the DGF locking members 81. Figures 31A - 31B schematically illustrates positioning the valve delivery sheath tip 202 at the native mitral valve. It is contemplated that the prosthetic valve 34 deployment procedure can occur while rapid pacing is in place. In one example view, shown in Figures 31A - 31B, the valve delivery system 131 can be selectively pushed outside the distal end of the docking sheath 94 so that the valve sheath 201 is positioned next to the tip of the posterior leaflet 4 or deep into the left ventricle 2.

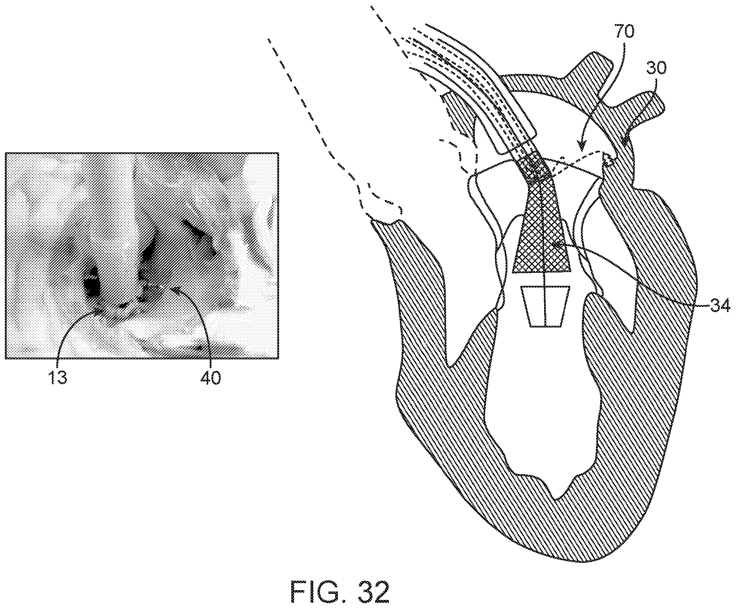

Figure 32 schematically shows the valve deployment process when the prosthetic valve 34 has been nearly completely released from the valve delivery system 131. In some cases, the valve sheath cap 271 has been advanced into the left ventricle 2 to release the lower ventricular portion of the stent 42, and the valve sheath 201 is incrementally retracted towards the docking sheath 92 to release the upper flared portion of the stent 41.

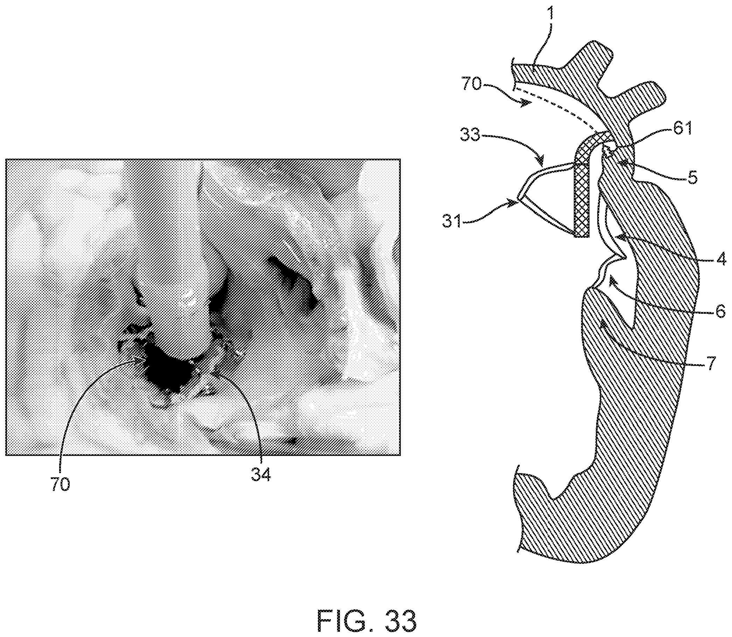

Once the prosthetic valve 34 is fully released from the valve delivery system 131, the atrial flared portion of the stent 41 can operatively situate on top of the native annulus 5, as illustrated in Figure 33.

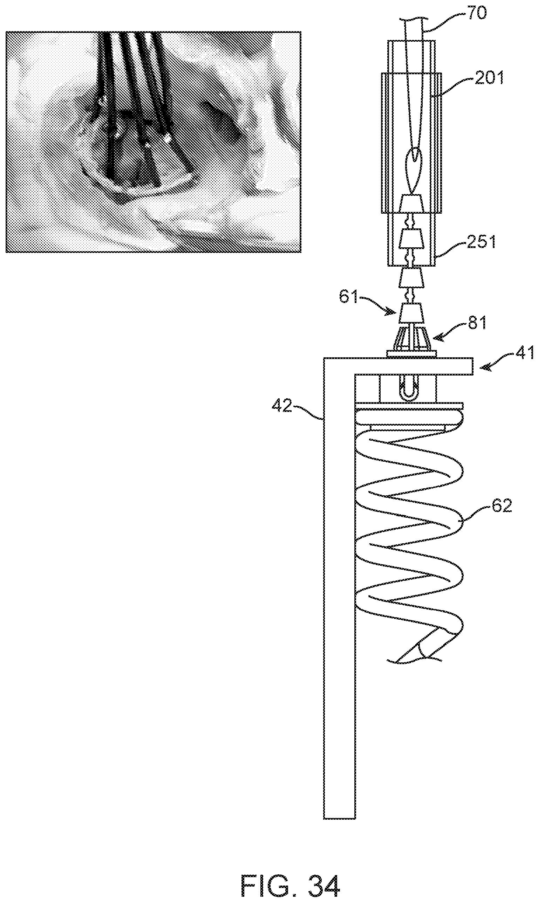

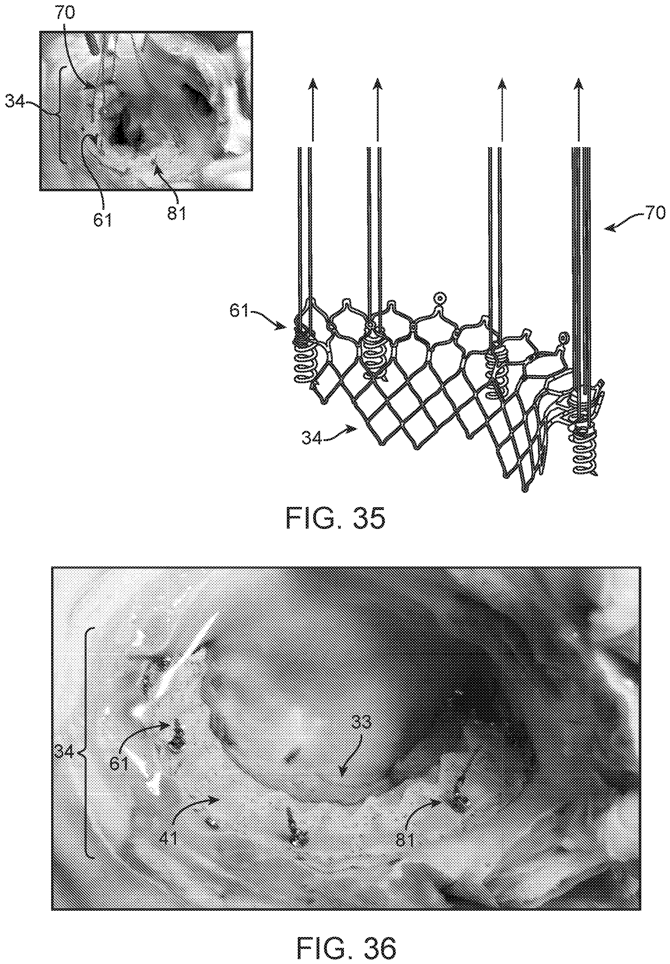

Following full release of the valve, the prosthetic valve 34 is locked into place at the DGF head members 62 by deploying the DGF locking members 81 as schematically shown in Figure 34. The DGF tail members 70 are then sequentially removed from the body as schematically shown in Figure 35.

The efficacy of the heart valve leaflet replacement system 47 implanted in an *ex vivo* pig heart by the MSML delivery system was tested by pressurizing the left ventricle 2. The prosthetic leaflets 33 were able to coapt with the native anterior mitral leaflet 3 as shown in Figure 36 without leakage. The heart valve leaflet replacement system 47 was stable within the pig heart under pressurization.

In some cases, it is contemplated that a two-step deployment procedure can be implemented to release the crescent shaped prosthetic valve 34 into the normal functional configuration. In the first step, the prosthetic valve 34 can be deployed into the left ventricle 2 at the level of the native mitral annulus 5, during which the prosthetic valve 34 can maintain a full cylindrical shape with the conjoining mechanism 46 of the two ends still in place. The prosthetic valve 34 can be oriented in a way that the markers on the stent structure can correspond to the two trigones of the native mitral annulus. In the second step, the conjoining mechanism 46 of the two ends of the prosthetic valve 34 can be released and the prosthetic valve 34 can be deployed into the designed semi-cylindrical shape to replace the native posterior mitral leaflet 4.

Subsequently, the prosthetic valve 34 can be locked in position relative to the native annulus 5 by locking the atrial flared portion of the stent 41 at the stent openings 44 to the DGF members 61. By tensioning the DGF member tails 70 during locking of the prosthetic valve 34 in place by the implanting DGF member bodies 64, the native annulus can be reshaped to the prosthetic valve 34 shape. It is contemplated that the means for locking the prosthetic valve 34 to the DGF members 61 can provide flexibility so that the prosthetic valve 34 can be locked in place at the annulus 5 even when the DGF members 61 are implanted in a non-optimal configuration, i.e., when the DGF members 61 are unevenly spaced or out of plane from each other on the annulus 5.

In some cases, the prosthetic valve 34 can be locked to the DGF members 61 simultaneously using the MSML delivery system. Tensioning the DGF member tails 70 while advancing the DGF locking member sheath 251 can further reduce the annulus 5 and allow the annulus 5 to be cinched radially after locking the prosthetic valve 34 in place.

In some cases, it is contemplated that following deployment of the heart valve leaflet replacement system, should there be any paravalvular leak or instability of the prosthetic valve 34 in the operative position, a plurality of additional DGF members 61 can optionally be deployed on top of the heart valve leaflet replacement system 47, such that the head portion of the DGF member 62 is driven through the atrial flared portion of the stent 41 and embedded into the muscular annulus tissue 5 until the body portion of the DGF member 64 lies flush against the atrial flared portion of the stent 41.

It is contemplated that the MSML delivery system can be configured to be mounted on a T-slotted bar, such that the lateral and angular positioning of the MSML delivery system can be adjusted to the desirable positioning to facilitate deployment of the heart valve leaflet replacement system 47.

In some cases, the method of delivering the heart valve leaflet replacement system 47 can be based on optional delivery access approaches. In some cases, the method can entail a trans-septal access approach. In some cases, an opening can be created in the internal jugular vein for the subsequent minimally invasive delivery of portions of the heart valve leaflet replacement system 47 through the superior vena cava 9 which flows into the right atrium of the heart. In this some cases, the delivery path crosses the atrial septum 11 of the heart, and once achieved, the MSML delivery system can operatively access the left atrium 1, the native mitral valve, and the left ventricle 2. In some cases, it is contemplated that the delivery path to the native mitral valve can be accessed trans-septally via a formed opening in the femoral vein, or the delivery path to the native mitral valve can be done trans-apically. In some cases, it is contemplated that the MSML delivery system can be placed along the delivery path to allow all of the heart valve leaflet replacement system 47 components needed for the implant procedure to enter the left atrium 1 without complications.

In some cases, the heart valve leaflet replacement system 47 can comprise an optional means for cinching the mitral annulus 5 circumferentially is shown in Figure 31. This means for cinching can entail a cinching tether that extends circumferentially along the mitral annulus. In some cases, an additional cinching tether can be pre-attached to the atrial flared portion of the stent 41. Optionally, an additional cinching tether can be pre-attached to the DGF member(s) 61. Operationally, once the prosthetic valve 34 is locked to the implanted DGF members 61, the cinching procedure can be carried out. In one embodiment, the means for locking the cinching tether can be of a similar design to the means for locking the prosthetic valve 34 to the DGF members 61.

In some cases, the methodology of delivering the heart valve leaflet replacement system 47 can further comprise occluding the formed opening in the atrial septum 11 if necessary. Once the septum 11 is successfully closed, the MSML delivery system can be removed from the body and the procedure is complete.

It is further contemplated that an accessory crimping device may be specially designed to crimp the prosthetic valve which aids in the uniform crimping of the prosthetic valve and prevents tangling or overlapping of the DGF tail members 70.

## Claims

1. A multi-stage and multi-lumen (MSML) delivery system for implanting a prosthetic heart valve comprising a valve stent (141) for treatment of a diseased heart valve, the MSML delivery system comprising:
a docking system (91) comprising a docking sheath (94) and a docking controller;
a dual-guiding-and-fixation (DGF) system for implanting a plurality of DGF members (61),
**characterised in that**:
the DGF system comprising a DGF sheath , a torque-driving shaft and a DGF controller; wherein the plurality DGF members (61) are configured to couple to a flared portion of the valve stent (141), and
a valve delivery system for releasing and locking the prosthetic heart valve, the valve delivery system comprising a valve sheath (201), a lock housing structure sheath and a plurality of DGF locking member sheaths (251), a valve stabilization mechanism (243), and a valve delivery controller;
wherein the docking system (91), the DGF system, and the valve delivery system are collectively configured to advance a plurality of DGF head members (62) to an operative position, to deliver and implant the plurality of DGF head members (62) to the operative position, and to guide, deliver and fixate the prosthetic heart valve to the operative position.

2. The MSML delivery system of claim 1, wherein each of the plurality of DGF members (61) comprises a DGF body member (64) and a DGF head member (62), wherein the torque-driving shaft is configured to fit inside a lumen of the DGF sheath, engage the DGF body member (64) and drive the DGF head member (62) into a targeted tissue; and wherein the torque-driving shaft is configured to bend together with the DGF sheath at the distal end during a DGF member delivery.

3. The MSML delivery system of claim 2, wherein the torque-driving shaft comprises a distal tip that is configured to engage the engager portion of the DGF body member (64); wherein each of the plurality of DGF members further comprises a DGF tail member (70) extended from the DGF body member (64), wherein an inner lumen of the DGF sheath is configured to house the DGF tail member (70), and wherein the DGF tail member (70) is configured to couple with a grabber (107) at the proximal end of the DGF system to fixate the DGF tail member (70) during the DGF member delivery.

4. The MSML delivery system of claim 1, wherein each of the plurality of DGF members further comprises a DGF tail member (70) extended from the DGF body member (64), wherein the DGF system further comprises an expandable compartmentalization sheath (121) with a plurality of lumens to organize the DGF tail member (70) during a DGF member delivery; wherein the inner walls separating the plurality of lumens are flexible, bendable, and collapsible, and are configured to be pushed to either side to allow a passage of other members of the DGF system during the DGF member delivery.

5. The MSML delivery system of claim 1, wherein each of the plurality of DGF members comprises a DGF head member (62), a DGF body member (64), a DGF tail member (70), a DGF locking unit (67), and a DGF locking member (81).

6. The MSML delivery system of claim 5, wherein the DGF head member (62) is configured to have a spiral, coil, barb, or hook shape, or other shapes that are configured to engage heart tissues.

7. The MSML delivery system of claim 5, wherein the DGF body member (64) is configured to link to the DGF head member (62), wherein the DGF body member (64) comprises (i) an engager for selectively engaging and disengaging a torque-driving shaft configured to embed the DGF head member (62) into an annular tissue, and (ii) a fixation mechanism for locking the prosthetic heart valve to the implanted DGF members.

8. The MSML delivery system of claim 7, wherein the engager of the DGF body member (64) is comprises a male protrusion, and wherein the male protrusion is configured to engage a recess with a corresponding shape on the distal tip of the torque-driving shaft.

9. The MSML delivery system of claim 5, wherein the DGF tail member (70) comprises an elongated tether that is configured to guide the prosthetic heart valve to the DGF body members (64) implanted in position, and wherein the elongated tether extends from the DGF body member (64) to the proximal end of the delivery system.

10. The MSML delivery system of claim 9, wherein the DGF tail member (70) is configured to attach to or detach from the DGF body member (64) by forming a loop shape at the proximal portion of the DGF body member (64) such that the DGF tail member (70) is looped pass through, and then is selectively removed by pulling one end.

11. The MSML delivery system of claim 5, wherein the DGF locking member (81) is configured to accept passage of the DGF tail member (70), and wherein the DGF locking member (81) is configured to pass through the DGF locking units (67) on the DGF body member (64) in one direction only, and the DGF locking member (81) is further configured not to pass through the prosthetic heart valve.

12. The MSML delivery system of claim 5, wherein one pair of one locking unit and one locking member are configured to become one locked piece, and the pair of one locking unit and one locking member is configured to attach directly to the flared portion of the valve stent.

13. The MSML delivery system of claim 5, wherein the DGF locking member (81) is configured to be a hollow conical shape with a plurality of teeth rising from a circular base that is open up to allow one-way passage of a specially designed locking unit on the DGF body member (64), wherein the plurality of teeth are configured to be snapped shut to engage with the DGF locking units (67) on the DGF body member.

14. The MSML, delivery system of claim 1, wherein each of the plurality of DGF members comprises a DGF locking member (81) and a DGF tail member, and wherein the lock housing structure sheath comprises a plurality of lumens for separating the DGF locking member (81) and the DGF tail member (70).

15. The MSML delivery system of claim 1, wherein the valve stabilization mechanism (243) comprises: a valve stabilization mechanism tube (278) running through the length of the valve delivery system, a valve stabilization mechanism tether that is looped through portions of the valve stent and through the valve stabilization mechanism tube (278) to the proximal end of the valve delivery system, and a grabber (107) to selectively fixate the two free ends of the valve stabilization mechanism tether.

## Patentansprüche

1. Ein mehrstufiges und mehrlumiges (MSML) Zuführsystem für das Implantieren einer Herzklappenprothese, umfassend einen Herzklappen-Stent (141) für die Behandlung einer erkrankten Herzklappe, das MSML-Zuführsystem umfassend:
Ein Dockingsystem (91), das eine Dockinghülse (94) und eine Dockingsteuerung umfasst;
ein Doppelführungs- und Befestigungssystem (DGF) für das Implantieren einer Vielzahl von DGF-Elementen (61),
**dadurch gekennzeichnet, dass**:
das DGF-System eine DGF-Hülse, eine Drehmoment-Antriebswelle und eine DGF-Steuerung umfasst; wobei die Vielzahl der DGF-Elemente (61) konfiguriert ist, einen aufgebördelten Abschnitt des Herzklappen-Stents (141) zu koppeln, und
ein Herzklappen-Zuführsystem für das Lösen und Sichern der Herzklappenprothese, das Herzklappen-Zuführsystem umfassend eine Herzklappenhülse (201), eine Verschlussgehäuse-Strukturhülse und eine Vielzahl von DGF-Verschlusselement-Hülsen (251), einen Herzklappen-Stabilisierungsmechanismus (243) und eine Herzklappen-Zuführsteuerung;
wobei das Dockingsystem (91), das DGF-System und das Herzklappen-Zuführsystem kollektiv so konfiguriert sind, dass sie eine Vielzahl von DGF-Kopfelementen (62) in eine Betriebsposition führen, um die Vielzahl der DGF-Kopfelemente (62) in die Betriebsposition zu setzen und zu implantieren, und die Herzklappenprothese in die Betriebsposition zu führen, einzusetzen und zu fixieren.

2. MSML-Zuführsystem nach Anspruch 1, wobei jedes der Vielzahl von DGF-Elementen (61) ein DGF-Körperelement (64) und ein DGF-Kopfelement (62) umfasst, wobei die Drehmoment-Antriebswelle so konfiguriert ist, dass sie in ein Lumen der DGF-Hülse passt, sich mit dem DGF-Körperelement (64) verbindet und das DGF-Kopfelement (62) in ein Zielgewebe treibt; und wobei die Drehmoment-Antriebswelle so konfiguriert ist, dass sie sich zusammen mit der DGF-Hülse am distalen Ende während einer Zuführung eines DGF-Elements biegt.

3. Das MSML-Zuführsystem nach Anspruch 2, wobei die Drehmoment-Antriebswelle eine distale Spitze umfasst, die konfiguriert ist, das Eingreifelement des DGF-Körperelements (64) einrasten zu lassen; wobei jedes der Vielzahl von DGF-Elementen fernen ein DGF-Endelement (70) umfasst, das sich von dem DGF-Körperelement (64) erstreckt, wobei ein Innenlumen der DGF-Hülse konfiguriert ist, das DGF-Endelement (70) aufzunehmen, und wobei das DGF-Endelement (70) konfiguriert ist, sich mit einem Greifer (107) an dem proximalen Ende des DGF-Systems zu koppeln, um das DGF-Endelement (70) während der Zuführung des DGF-Elements zu fixieren.

4. Das MSML-Zuführsystem nach Anspruch 1, wobei jedes der Vielzahl von DGF-Elementen ferner ein DGF-Endelement (70) umfasst, das sich von dem DGF-Körperelement (64) erstreckt, wobei das DGF-System ferner eine expandierbare Kompartimentierungs-Hülse (121) mit einer Vielzahl von Lumen umfasst, um das DGF-Endelement (70) während einer DGF-Element-Zuführung zu organisieren; wobei die Innenwände, die die Vielzahl von Lumen trennen, flexibel, biegbar und faltbar sind und so konfiguriert, dass sie auf jede Seite gedrückt werden können, um einen Durchgang von anderen Elementen des DGF-Systems während der Zuführung des DGF-Elements zu erlauben.

5. Das MSML-Zuführsystem nach Anspruch 1, wobei jedes der Vielzahl von DGF-Elementen ein DGF-Kopfelement (62), ein DGF-Körperelement (64), und ein DGF-Endelement (70), eine DGF-Verschlusseinheit (67) und ein DGF-Verschlusselement (81) umfasst.

6. Das MSML-Zuführsystem nach Anspruch 5, wobei das DGF-Kopfelement (62) mit einer Spiral-, Spulen-, Stachel- oder Hakenform oder anderen Formen konfiguriert ist, die konfiguriert sind, in das Herzgewebe einzugreifen.

7. Das MSML-Zuführsystem nach Anspruch 5, wobei das DGF-Körperelement (64) konfiguriert ist, das DGF-Kopfelement (62) zu verbinden, wobei das DGF-Kopfelement (64) (i) ein Eingreifelement für das selektive Eingreifen und Auslösen einer Drehmoment-Antriebswelle umfasst, die konfiguriert ist, das DGF-Kopfelement (62) in ein ringförmiges Gewebe einzubetten, und (ii) einen Befestigungsmechanismus für das Verriegeln der Herzklappenprothese an den implantierten DGF-Elementen umfasst.

8. Das MSML-Zuführsystem nach Anspruch 7, wobei das Eingreifelement des DGF-Körperelements (64) einen Vorsprung umfasst, und wobei der Vorsprung konfiguriert ist, in eine Vertiefung mit einer entsprechenden Form an der distalen Spitze der Drehmoment-Antriebswelle einzugreifen.

9. Das MSML-Zuführsystem nach Anspruch 5, wobei das DGF-Endelement (70) ein längliches Halteband umfasst, das konfiguriert ist, die Herzklappenprothese zu den in der Position implantierten DGF-Körperelementen (64) zu führen, und wobei sich das längliche Halteband von dem DGF-Körperelement (64) zu dem proximalen Ende des Zuführsystems erstreckt.

10. Das MSML-Zuführsystem nach Anspruch 9, wobei das DGF-Endelement (70) konfiguriert ist, sich mit dem DGF-Körperelement (64) durch Bilden einer Schleifenform an dem proximalen Abschnitt des DGF-Körperelements (64) zu verbinden oder davon zu lösen, so dass das DGF-Endelement (70) in einer Schleife hindurchgeführt und dann selektiv durch Ziehen an einem Ende entfernt wird.

11. Das MSML-Zuführsystem nach Anspruch 5, wobei das DGF-Verschlusselement (81) konfiguriert ist, den Durchgang des DGF-Endelements (70) zu akzeptieren, und wobei das DGF-Verschlusselement (81) konfiguriert ist, durch die DGF-Verschlusseinheiten (67) an den DGF-Körperelementen (64) in nur eine Richtung hindurchzugehen, und das DGF-Verschlusselement (81) ferner konfiguriert ist, nicht durch die Herzklappenprothese zu gehen.

12. Das MSML-Zuführsystem nach Anspruch 5, wobei ein Paar einer Verschlusseinheit und ein Verschlusselement konfiguriert sind, ein Verschlussteil zu werden, und das Paar einer Verschlusseinheit und eines Verschlusselements konfiguriert ist, sich direkt mit dem aufgebördelten Abschnitt des Herzklappen-Stents zu verbinden.

13. Das MSML-Zuführsystem nach Anspruch 5, wobei das DGF-Verschlusselement (81) als hohle, konische Form mit einer Vielzahl von Zähnen konfiguriert ist, die sich aus einer kreisförmigen Basis nach oben erstrecken, die offen ist, um einen Durchgang in eine Richtung einer speziell konzipierten Verschlusseinheit an dem DGF-Körperelement (64) zu erlauben, wobei die Vielzahl von Zähnen konfiguriert ist, zuzuschnappen, um in die DGF-Verschlusseinheiten (67) an dem DGF-Körperelement einzugreifen.

14. Das MSML-Zuführsystem nach Anspruch 1, wobei jedes der Vielzahl von DGF-Elementen ein DGF-Verschlusselement (81) und ein DGF-Endelement umfasst und wobei die Hülse der Verschlussgehäusestruktur eine Vielzahl von Lumen für die Trennung des DGF-Verschlusselements (81) und des DGF-Endelements (70) umfasst.

15. Das MSML-Zuführsystem nach Anspruch 1, wobei der Herzklappen-Stabilisierungsmechanismus (243) Folgendes umfasst: Einen Schlauch des Herzklappen-Stabilisierungsmechanismus (278), der durch die Länge des Herzklappen-Zuführsystems verläuft, ein Halteband des Herzklappen-Stabilisierungsmechanismus, das als Schleife durch die Abschnitte des Herzklappen-Stents und durch den Schlauch des Herzklappen-Stabilisierungsmechanismus (278) zu dem proximalen Ende des Herzklappen-Zuführsystems geführt wird, und einen Greifer (107), um selektiv die beiden freien Enden des Haltebands des Herzklappen-Stabilisierungsmechanismus zu fixieren.

## Revendications

1. Système de délivrance multiétapes et multilumières (MSML) destiné à implanter une valvule cardiaque prothétique comprenant une endoprothèse valvulaire (141) pour le traitement d'une valvule cardiaque malade, le système de délivrance MSML comprenant :
un système d'amarrage (91) comprenant une gaine d'amarrage (94) et un contrôleur d'amarrage ;
un système à double guidage et fixation (DGF) destiné à implanter une pluralité d'éléments DGF (61),
**caractérisé en ce que** :
le système DGF comprend une gaine DGF, un arbre d'entraînement du couple et un contrôleur DGF ; dans lequel la pluralité d'éléments DGF (61) est conçue pour s'accoupler à une partie évasée de l'endoprothèse valvulaire (141), et
un système de délivrance de valvule destiné à libérer et à verrouiller la valvule cardiaque prothétique, le système de délivrance de valvule comprenant une gaine de valvule (201), une gaine de structure de logement de verrou et une pluralité de gaines d'élément de verrouillage DGF (251), un mécanisme de stabilisation de valvule (243), et un contrôleur de délivrance de valvule ;
dans lequel le système d'amarrage (91), le système DGF et le système de délivrance de valvule sont collectivement conçus pour faire avancer une pluralité d'éléments de tête DGF (62) jusqu'à une position fonctionnelle, pour délivrer et implanter la pluralité d'éléments de tête DGF (62) jusqu'à la position opérationnelle, et pour guider, délivrer et fixer la valvule cardiaque prothétique jusqu'à la position fonctionnelle.

2. Système de délivrance MSML selon la revendication 1, dans lequel chaque élément de la pluralité d'éléments DGF (61) comprend un élément de corps DGF (64) et un élément de tête DGF (62), dans lequel l'arbre d'entraînement du couple est conçu pour s'adapter à l'intérieur d'une lumière de la gaine DGF, entrer en prise avec l'élément de corps DGF (64) et entraîner l'élément de tête DGF (62) dans un tissu ciblé ; et dans lequel l'arbre d'entraînement du couple est conçu pour s'incurver conjointement à la gaine DGF à l'extrémité distale pendant la délivrance d'un élément DGF.

3. Système de délivrance MSML selon la revendication 2, dans lequel l'arbre d'entraînement du couple comprend une pointe distale qui est conçue pour entrer en prise dans la partie du mécanisme d'entrée en prise de l'élément de corps DGF (64) ; dans lequel chaque élément de la pluralité d'éléments DGF comprend en outre un élément de queue DGF (70) étendu à partir de l'élément de corps DGF (64), dans lequel une lumière intérieure de la gaine DGF est conçue pour loger l'élément de queue DGF (70), et dans lequel l'élément de queue DGF (70) est conçu pour s'accoupler avec un dispositif de saisie (107) à l'extrémité proximale du système DGF pour fixer l'élément de queue DGF (70) pendant la délivrance de l'élément DGF.

4. Système de délivrance MSML selon la revendication 1, dans lequel chaque élément de la pluralité d'éléments DGF comprend en outre un élément de queue DGF (70) étendu à partir de l'élément de corps DGF (64), dans lequel le système DGF comprend en outre une gaine de compartimentation extensible (121) comportant une pluralité de lumières pour organiser l'élément de queue DGF (70) au cours de la délivrance d'un élément DGF ; dans lequel les parois internes séparant la pluralité de lumières sont flexibles, pliables et repliables et sont conçues pour être poussées d'un côté ou de l'autre afin de permettre le passage d'autres éléments du système DGF pendant la délivrance d'un élément DGF.

5. Système de délivrance MSML selon la revendication 1, dans lequel chaque élément de la pluralité d'éléments DGF comprend un élément de tête DGF (62), un élément de corps DGF (64), un élément de queue DGF (70), une unité de verrouillage DGF (67) et un élément de verrouillage DGF (81).

6. Système de délivrance MSML selon la revendication 5, dans lequel l'élément de tête DGF (62) est conçu pour avoir une forme de spirale, d'enroulement, de barbelure ou de crochet, ou d'autres formes qui sont configurées pour entrer en prise avec les tissus cardiaques.

7. Système de délivrance MSML selon la revendication 5, dans lequel l'élément de corps DGF (64) est conçu pour être relié à l'élément de tête DGF (62), dans lequel l'élément de corps DGF (64) comprend (i) un mécanisme d'entrée en prise destiné à entrer en prise avec un arbre d'entraînement du couple et à se libérer de celui-ci de manière sélective, l'arbre est conçu pour intégrer l'élément de tête DGF (62) dans un tissu annulaire et (ii) un mécanisme de fixation destiné à verrouiller la valvule cardiaque prothétique avec les éléments DGF implantés.

8. Système de délivrance MSML selon la revendication 7, dans lequel le mécanisme d'entrée en prise de l'élément du corps DGF (64) comprend une protubérance mâle, et dans lequel la protubérance mâle est conçue pour entrer en prise dans un évidement de forme correspondante sur l'embout distal de l'arbre d'entraînement du couple.

9. Système de délivrance MSML selon la revendication 5, dans lequel l'élément de queue DGF (70) comprend une attache allongée conçue pour guider la valvule cardiaque prothétique vers les éléments de corps DGF (64) implantés en position et dans lequel l'attache allongée s'étend depuis l'élément de corps DGF (64) jusqu'à l'extrémité proximale du système de délivrance.

10. Système de délivrance MSML selon la revendication 9, dans lequel l'élément de queue DGF (70) est conçu pour s'attacher ou se détacher de l'élément de corps DGF (64) en constituant une forme de boucle à la partie proximale de l'élément de corps DGF (64) de sorte que l'élément de queue DGF (70) fait une boucle à travers, puis est retiré de manière sélective en tirant sur une extrémité.

11. Système de délivrance MSML selon la revendication 5, dans lequel l'élément de verrouillage DGF (81) est conçu pour accepter le passage de l'élément de queue DGF (70), et dans lequel l'élément de verrouillage DGF (81) est conçu pour passer à travers les unités de verrouillage DGF (67) sur l'élément de corps DGF (64) dans une seule direction et l'élément de verrouillage DGF (81) est conçu en outre pour ne pas passer à travers la valvule cardiaque prothétique.

12. Système de délivrance MSML selon la revendication 5, dans lequel une paire d'une unité de verrouillage et un élément de verrouillage sont conçus pour devenir une seule pièce verrouillée et la paire d'une unité de verrouillage et un élément de verrouillage sont conçus pour s'attacher directement à la partie évasée de l'endoprothèse valvulaire.

13. Système de délivrance MSML selon la revendication 5, dans lequel l'élément de verrouillage DGF (81) est conçu pour être de forme conique creuse comportant une pluralité de dents s'élevant à partir d'une base circulaire ouverte pour permettre le passage à sens unique d'une unité de verrouillage spécialement conçue sur l'élément de corps DGF (64), dans lequel la pluralité de dents est conçue pour être fermée par encliquetage pour entrer en prise avec les unités de verrouillage DGF (67) sur l'élément de corps DGF.

14. Système de délivrance MSML selon la revendication 1, dans lequel chaque élément de la pluralité d'éléments DGF comprend un élément de verrouillage DGF (81) et un élément de queue DGF et dans lequel la gaine de la structure de logement de verrou comprend une pluralité de lumières destinées à séparer l'élément de verrouillage DGF (81) et l'élément de queue DGF (70)

15. Système de délivrance MSML selon la revendication 1, dans lequel le mécanisme de stabilisation de valvule (243) comprend : un tube de mécanisme de stabilisation de valvule (278) courant sur toute la longueur du système de délivrance de valvule, une attache du mécanisme de stabilisation de valvule qui fait une boucle à travers des parties de l'endoprothèse valvulaire et à travers le tube du mécanisme de stabilisation de valvule (278) jusqu'à l'extrémité proximale du système de délivrance de la valvule et un dispositif de saisie (107) pour fixer de manière sélective les deux extrémités libres de l' attache de mécanisme de stabilisation de valvule.
